# EUROPEAN PATENT APPLICATION

(11) **EP 4 349 808 A1**
(43) Date of publication of application: **10.04.2024**
(21) Application number: 22943198.6
(22) Date of filing: 27.05.2022
(51) Int. Cl.: C07C 57/30, C07C 57/46, C07C 57/40, C07D 209/00, H01L 31/0216

(54) **ORGANIC COMPOUND AND USE THEREOF, PASSIVATION FILM, SOLAR CELL AND ELECTRONIC DEVICE**

(71) Applicant: Contemporary Amperex Technology Co., Limited, Ningde, Fujian 352100 (CN)
(72) Inventor: LIANG, Weifeng, Ningde, Fujian 352100 (CN); YANG, Yifang, Ningde, Fujian 352100 (CN); HUANG, Zhihan, Ningde, Fujian 352100 (CN); LUAN, Bo, Ningde, Fujian 352100 (CN); CHEN, Changsong, Ningde, Fujian 352100 (CN); LIN, Xiangling, Ningde, Fujian 352100 (CN); GUO, Yongsheng, Ningde, Fujian 352100 (CN); CHEN, Guodong, Ningde, Fujian 352100 (CN); OUYANG, Chuying, Ningde, Fujian 352100 (CN)
(74) Representative: Gong, Jinping
(86) International application number: PCT/CN2022/095578
(87) International publication number: WO 2023/226000

(57) **Abstract**

The present application provides an organic compound and use thereof, a passivation film, a solar battery, and an electrical apparatus. The organic compound is as shown in formula (1) or is an oxyacid radical salt of the compound as shown in formula (1), wherein Ar is selected from any one of substituted or unsubstituted aryl with 6 to 50 ring atoms, substituted or unsubstituted heteroaryl with 5 to 50 ring atoms, or substituted arylamino as shown in formula (A); L is selected from acyclic alkylene with 1 to 10 carbon atoms; and R₁ is an oxyacid group. The organic compound is capable of improving photon-to-electron conversion efficiency and stability of a solar battery device.

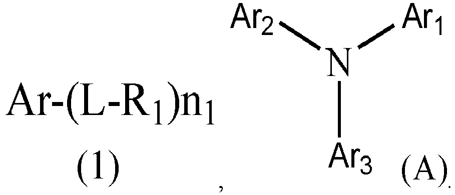

## Description

### TECHNICAL FIELD

The present application relates to the field of batteries, in particular to an organic compound and use thereof, a passivation film, a solar battery, and an electrical apparatus.

### BACKGROUND

Perovskite solar batteries have many features such as excellent photoelectric characteristics, high light absorption coefficient, long carrier life, and long diffusion length, and have become the best of the novel third-generation solar batteries. Among them, an inverted perovskite solar battery has attracted more and more attention for the advantages of good stability, low hysteresis effect etc. The inverted perovskite solar battery mainly includes a glass substrate as well as transparent conductive oxide (TCO), a hole transport layer HTL, a perovskite PVK layer, and an electron transport layer ETL that are sequentially disposed on the glass substrate.

Metal oxides such as nickel oxide are hole transport layer materials commonly used in the preparation of inverted perovskite solar batteries. However, nickel oxide will inevitably present trivalent nickel during preparation and use, whereas trivalent nickel will react with A-site cations such as formamidine ion (FA) and methylammonium ion (MA) present in perovskite to result in vacancy of A-site, so that excess lead halide remains at an interface between a perovskite layer and a hole transport layer, leading to excessive defects at the interface or unbalanced hole transport speed, increasing charge recombination at the interface, thereby reducing voltage at open circuit and fill factor of the solar battery, and in turn reducing photon-to-electron conversion efficiency and stability of the solar battery.

Therefore, the prior art is still to be improved.

### SUMMARY

In view of the above problems, the present application provides an organic compound and its use, a passivation film, a solar battery, and an electrical apparatus. The organic compound can improve photon-to-electron conversion efficiency and stability of a solar battery device.

In a first aspect, the present application provides an organic compound, the organic compound being as shown in formula (1): 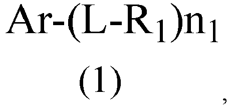 wherein Ar is selected from any one of substituted or unsubstituted aryl with 6 to 50 ring atoms, substituted or unsubstituted heteroaryl with 5 to 50 ring atoms, or substituted arylamino as shown in formula (A): 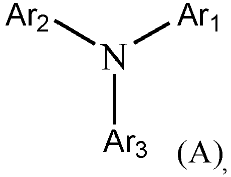
L is selected from acyclic alkylene with 1 to 10 carbon atoms; R₁ is an oxyacid group;
Ar₁ to Ar₃ are independently selected from any one of H, substituted or unsubstituted alkenyl with 2 to 30 carbon atoms, and substituted or unsubstituted aryl with 7 to 30 carbon atoms, respectively, and at least one of Ar₁ to Ar₃ is selected from substituted or unsubstituted aryl with 7 to 30 carbon atoms;
n₁ is selected from any integer from 1 to 3;
or the organic compound is an oxyacid radical salt of the compound as shown in formula (1).

In the technical solutions of the Examples of the present application, Ar, L, and R₁ with specific group structures are organically combined to form an organic compound of formula (1) or further form an oxyacid radical salt, wherein L is selected from acyclic alkylene with 1 to 10 carbon atoms and is connected with Ar and R₁ separately so as to form an organic whole, and R₁ is an oxyacid group, which may be combined with metal ions such as trivalent nickel so as to play a role of passivating the metal ions, while L plays a role of reducing steric hindrance. The organic compound formed by the organic combination of Ar, L and R₁ above may form an aggregate with an ordered structure through intermolecular interactions, has strong self-assembly ability so that a flat self-assembled passivation film can be prepared and the performance of the passivation film can be improved, and can improve photon-to-electron conversion efficiency and stability of a solar battery when used to prepare the solar battery.

Further, when the above-mentioned organic compound is applied to prepare a passivation film for a solar battery, on the one hand, regulating the number of carbon atoms in L may improve hydrophobicity of the passivation film, so as to further inhibit the negative effects of water and oxygen on perovskite light-absorbing material; on the other hand, the specific group structure in Ar enables the organic compound to have an energy level adapted to other functional layer materials in the solar battery, so that the photoconversion efficiency and stability of the solar battery may be further improved.

In some of the Examples, L is selected from acyclic alkylene with 3 to 11 carbon atoms.

Regulating the number of carbon atoms in L improves the hydrophobicity of the organic compound while reducing the steric hindrance of the organic compound, which further improves the conversion efficiency and stability of the solar battery.

In some of the Examples, the acyclic alkylene is linear alkylene.

In some of the Examples, R₁ is, at each occurrence, independently selected from any one of a phosphonic acid group, a sulfonic acid group, a carboxylic acid group, a sulfinic acid group, a boric acid group, and a silicic acid group, respectively.

In some of the Examples, R₁ is, at each occurrence, independently selected from any one of the following structures respectively: 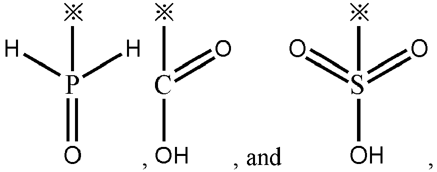 and 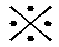 represents a linking site.

In some of the Examples, heteroatoms in the substituted or unsubstituted heteroaryl with 5 to 50 ring atoms are selected from at least one of N, O, and S.

In some of the Examples, Ar is selected from any one of formulas (A) to (C): 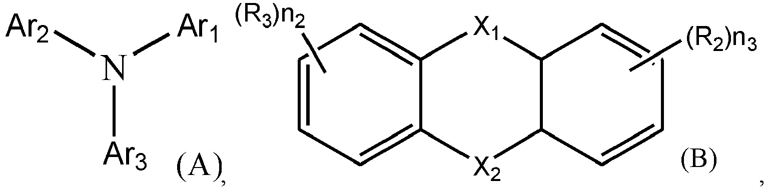 and 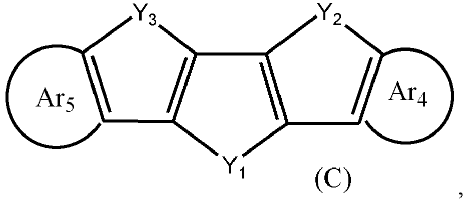
wherein X₁ and X₂ are independently selected from any one of a single bond, C(R₄R₅), O, S, N, NR₄, C=O, or S=O, respectively, and X₁ and X₂ are not single bonds at the same time;
Y₁, Y₂, and Y₃ are independently selected from any one of C(R₄R₅), O, S, N, NR₄, C=O, or S=O, respectively;
R₂ to R₅ are, at each occurrence, independently selected from any one of H, D, a halogen group, -N(R₆)₂, -CONR₆, -OCOR₆, substituted or unsubstituted alkyl with 1 to 30 carbon atoms, substituted or unsubstituted aryl with 6 to 30 carbon atoms, and substituted or unsubstituted heteroaryl with 5 to 30 carbon atoms, respectively;
R₆ is, at each occurrence, independently selected from any one of H, D, substituted or unsubstituted alkyl with 1 to 30 carbon atoms, substituted or unsubstituted alkenyl with 2 to 30 carbon atoms, substituted or unsubstituted aryl with 6 to 30 carbon atoms, and substituted or unsubstituted heteroaryl with 5 to 30 carbon atoms, respectively;
Ar₄ and Ar₅ are independently selected from any one of H, substituted or unsubstituted aryl with 6 to 30 carbon atoms, and substituted or unsubstituted heteroaryl with 5 to 30 carbon atoms, respectively;
n₂ and n₃ are independently selected from any integer from 1 to 4, respectively, with 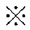 representing a linking site.

In some of the Examples, X₁ and X₂ are independently selected from any one of a single bond, C(R₄R₅), O, S, N, NR₄, respectively, and X₁ and X₂ are not single bonds at the same time;
Y₁, Y₂, and Y₃ are independently selected from any one of C(R₄R₅), O, S, and N, respectively.

In some of the Examples, R₂ to R₅ are, at each occurrence, independently selected from any one of H, D, a halogen group, amino, -CONR₆, -OCOR₆, unsubstituted alkyl with 1 to 5 carbon atoms, halogen- or carboxy-substituted alkyl with 1 to 5 carbon atoms, unsubstituted aryl with 6 to 15 carbon atoms, halogen-substituted aryl with 6 to 15 carbon atoms, unsubstituted heteroaryl with 6 to 15 carbon atoms, and halogen-substituted heteroaryl with 5 to 15 carbon atoms, respectively;

R₆ is, at each occurrence, independently selected from any one of H, D, substituted or unsubstituted alkyl with 1 to 5 carbon atoms, substituted or unsubstituted alkenyl with 2 to 6 carbon atoms, substituted or unsubstituted aryl with 6 to 15 carbon atoms, and substituted or unsubstituted heteroaryl with 5 to 15 carbon atoms, respectively.

In some of the Examples, R₄ in NR₄ is, at each occurrence, independently selected from any one of H, D, substituted or unsubstituted alkyl with 1 to 5 carbon atoms, and the following structures, respectively: 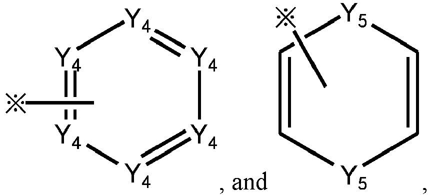
Y₄ is, at each occurrence, independently selected from CR₉ or N, respectively, and Y₄ in the same structural formula is not N at the same time;
Y₅ is, at each occurrence, independently selected from CR₉R₁₀ or NR₉, respectively;
R₉ to R₁₀ are, at each occurrence, independently selected from any one of H, D, substituted or unsubstituted linear alkyl with 1 to 10 carbon atoms, substituted or unsubstituted branched alkyl with 3 to 20 carbon atoms and substituted or unsubstituted cycloalkyl with 3 to 20 carbon atoms, respectively; 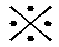
represents a linking site with N in NR₄.

In some of the Examples, Ar₄ and Ar₅ are independently selected from any one of H and the following structures, respectively: 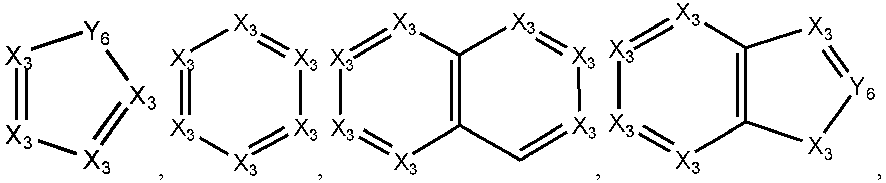 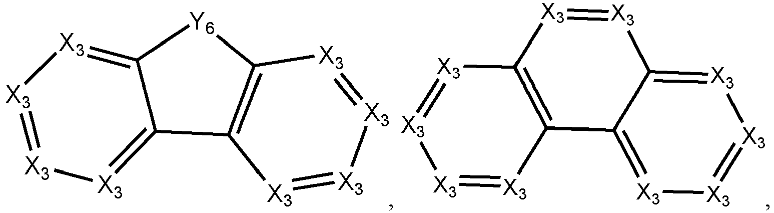 and 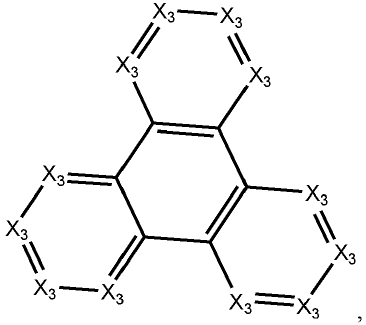
wherein: Y₆ is selected from any one of CR₇R₈, O, S, S=O, and C=O;
X₃ is, at each occurrence, independently selected from CR₇ or N, respectively, and X₃ in the same structural formula is not N at the same time;
R₇ and R₈ are, at each occurrence, independently selected from H, D, substituted or unsubstituted linear alkyl with 1 to 10 carbon atoms, substituted or unsubstituted branched alkyl with 3 to 20 carbon atoms and substituted or unsubstituted cycloalkyl with 3 to 20 carbon atoms, respectively.

In some of the Examples, Ar is selected from any one of following structures: 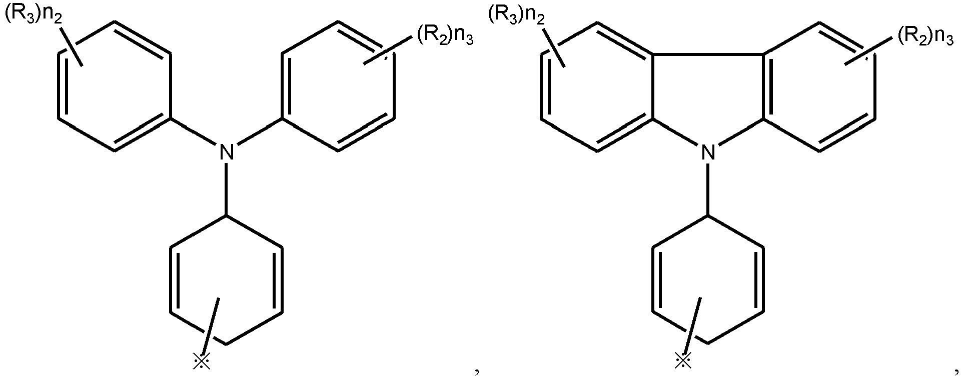 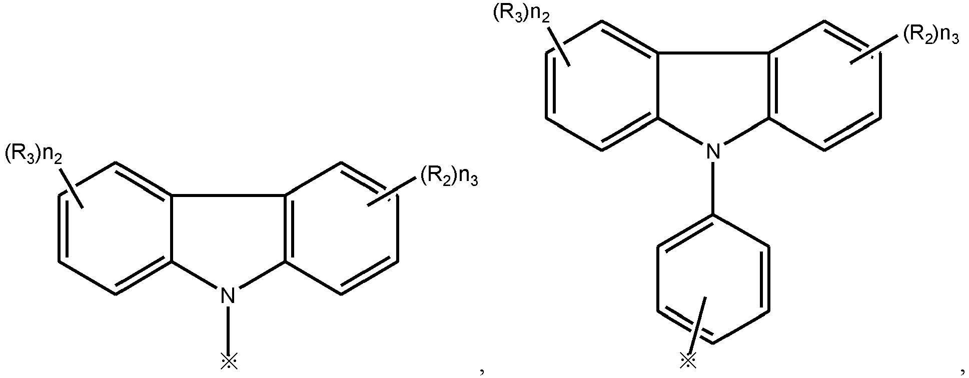 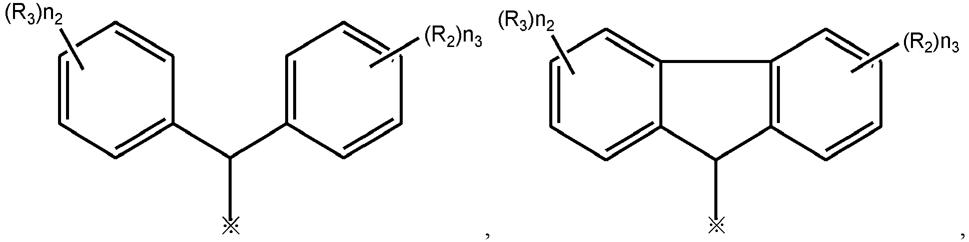 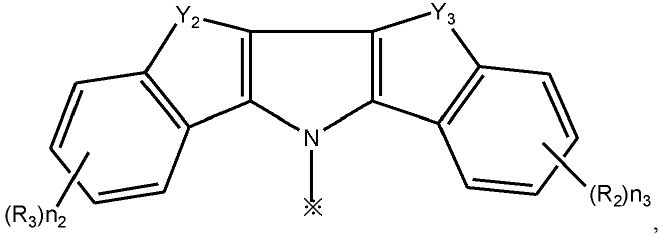 and

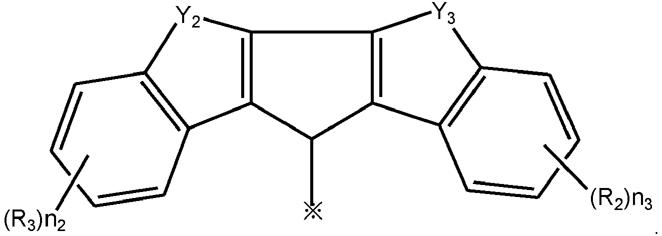

In some of the Examples, the oxyacid radical salt of the compound as shown in formula (1) comprises an anion and a cation, the anion is formed by at least one alcoholic hydroxyl in an oxyacid group in the compound as shown in formula (1) losing H, and the cation is selected from a metal ion or NH₄⁺.

In a second aspect, the present applicaiton provides use of an organic compound as a metal deactivator , the organic compound being as shown in formula (1): 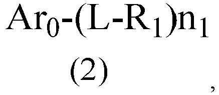
wherein Arc is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group;
n₁ is selected from any integer from 1 to 3;
or the organic compound is an oxyacid radical salt of the compound as shown in formula (2).

In a third aspect, the present application provides use of the above-mentioned organic compound in the preparation of a passivation film, the organic compound being as shown in formula (2): 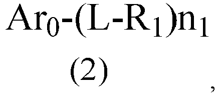
wherein Arc is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group;
n₁ is selected from any integer from 1 to 3;
or the organic compound is an oxyacid radical salt of the compound as shown in formula (2).

In a fourth aspect, the present application provides a passivation film, components of which comprise the organic compound as shown in formula (2) and/or the oxyacid radical salt of the compound as shown in formula (2): 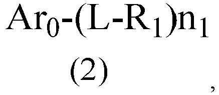
wherein Arc is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group;
n₁ is selected from any integer from 1 to 3.

In a fifth aspect, the present application provides a method for preparing the passivation film of the fourth aspect, comprising the following steps:
mixing the organic compound as shown in formula (2) and/or the oxyacid radical salt of the compound as shown in formula (2) and a solvent to prepare a mixed solution;
and coating a surface of a substrate with the mixed solution, and drying to obtain a passivation film.

In a sixth aspect, the present application provides a solar battery, wherein the solar battery comprises a hole transport layer and a passivation layer that are stacked, components of the hole transport layer comprise a metal oxide, and the passivation layer comprise the organic compound as shown in formula (2) and/or the oxyacid radical salt of the compound as shown in formula (2): 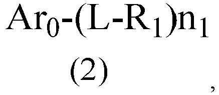
wherein Arc is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group;
n₁ is selected from any integer from 1 to 3.

In a seventh aspect, the present application provides an electrical apparatus, comprising the solar battery provided in the sixth aspect.

The above description is only an overview of the technical solutions of the present application. In order to more clearly understand the technical means of the present application to implement same according to the contents of the description, and in order to make the above and other objectives, features and advantages of the present application more obvious and understandable, specific embodiments of the present application are exemplarily described below.

### BRIEF DESCRIPTION OF THE DRAWINGS

Various other advantages and benefits will become apparent to those of ordinary skill in the art upon reading the following detailed description of the preferred embodiments. The drawings are for the purpose of illustrating the preferred embodiments only and are not to be considered a limitation to the present application. Furthermore, in all the drawings, the same reference numerals represent the same components. In the drawings:
FIG. 1 is a schematic diagram of a solar battery according to an embodiment of the present application;
FIG. 2 is a schematic diagram of an electrical apparatus according to an embodiment of the present application in which a solar battery is used as a power source.

List of reference numerals:
10 - solar battery; 11 - first electrode; 12 - hole transport layer; 13 - passivation layer; 14 - perovskite layer; 15 - electron transport layer; 16 - buffer layer; 17 - second electrode; and 20 - electrical apparatus.

### DETAILED DESCRIPTION OF THE EMBODIMENTS

Examples of the technical solutions of the present application will be described in detail below in conjunction with the drawings. The following Examples are only used to more clearly illustrate the technical solutions of the present application, and therefore are only used as examples and cannot be used to limit the scope of protection of the present application.

Unless otherwise defined, all technical and scientific terms used herein have the same meaning as commonly understood by those skilled in the art belonging to the technical field of the present application; the terms used herein are intended only for the purpose of describing specific Examples and are not intended to limit the present application; the terms "including" and "having" and any variations thereof in the Specification and the Claims of the present application and in the description of drawings above are intended to cover non-exclusive inclusion.

In the description of the Examples of the present application, the technical terms "first", "second", and the like are used only to distinguish between different objects, and are not to be understood as indicating or implying a relative importance or implicitly specifying the number, particular order, or primary and secondary relation of the technical features indicated. In the description of the Examples of the present application, the meaning of "a plurality of" is two or more, unless otherwise explicitly and specifically defined.

Reference herein to "an Example" means that a particular feature, structure, or characteristic described in connection with the Example can be included in at least one Example of the present application. The appearance of this phrase in various places in the Specification does not necessarily refer to the same Example, nor is it a separate or alternative Example that is mutually exclusive with other Examples. It is explicitly and implicitly understood by those skilled in the art that the Example described herein may be combined with other Examples.

In the description of the Examples of the present application, the term "and/or" is only an association relationship for describing associated objects, indicating that there may be three relationships, for example A and/or B may represent three situations: A exists alone, both A and B exist, and B exists alone. In addition, the character "/" herein generally represents that previous and next associated objects form an "or" relationship.

In the description of the Examples of the present application, the term "a plurality of" refers to two or more (including two), and similarly, "multiple groups" refers to two or more (including two) groups, and "multiple sheets" refers to two or more (including two) sheets.

In the description of the Examples of the present application, the orientation or position relationship indicated by the technical terms "center", "longitudinal", "transverse", "length", "width", "thickness", "upper," "lower," "front," "back," "left," "right," "vertical," "horizontal," "top," "bottom," "inner," "outer," "clockwise," "counterclockwise," "axial", "radial", "circumferential", etc. are based on the orientation or position relationship shown in the drawings and are intended to facilitate the description of the Examples of the present application and simplify the description only, rather than indicating or implying that the device or element referred to must have a particular orientation or be constructed and operated in a particular orientation, and therefore are not to be interpreted as limitations on the Examples of the present application.

In the description of the Examples of the present application, unless otherwise expressly specified and limited, the technical terms "mount," "join," "connect," "fix," etc. should be understood in a broad sense, such as, a fixed connection, a detachable connection, or an integral connection; a mechanical connection, or an electrical connection; a direct connection, an indirect connection through an intermediate medium, an internal connection of two elements, or interaction between two elements. For those of ordinary skill in the art, the specific meanings of the above terms in the Examples of the present application can be understood according to specific situations.

In the present invention, the term "alkyl" refers to a group formed by an alkane losing one hydrogen, such as methyl formed by methane losing one hydrogen; "alkylene or alkylene group" refers to a group formed by an alkane losing two hydrogens, such as methylene formed by methane losing two hydrogens.

The term "acyclic alkane" refers to an alkane in which all carbon atoms are connected via carbon-carbon single bonds and do not form a ring while the remaining valence bonds are combined with hydrogen, including linear alkanes and branched alkanes.

In the present invention, the number of carbon atoms in the "acyclic alkylene" may be 1 to 10, including 1, 2, 3, 4, 5, 6, 7, 8, 9, or 10, which means a group formed after an acyclic alkane containing 1 to 10 carbon atoms (i.e. C1-10 acyclic alkanes) loses two hydrogens, specific examples include a group formed after a C1 acyclic alkane, C2 acyclic alkane, C3 acyclic alkane, C4 acyclic alkane, C5 acyclic alkane, C6 acyclic alkane, C7 acyclic alkane, C8 acyclic alkane, C9 acyclic alkane, or C10 acyclic alkane loses two hydrogens, and non-limiting examples of "C1-10 acyclic alkanes" include methane, ethane, n-propane, isopropane, n-butane, isobutane, 2-ethylbutane, 3,3-dimethylbutane, n-pentane, isopentane, neopentane, 1-methylpentane, 3-methylpentane, 2-ethylpentane, 4-methyl-2-pentane, n-hexane, 1-methylhexane, 2-ethylhexane, 2-butylhexane, n-heptane, 1-methylheptane, 2,2-dimethylheptane, 2-ethylheptane, n-octane, n-nonane, and n-decane. In other words, non-limiting examples of "C1-10 acyclic alkylene" include groups formed after the above-mentioned alkanes lose two hydrogens.

In the present invention, the "number of ring atoms" represents the number of atoms bonded into a ring, and when the ring is substituted by a substituent, the atoms contained in the substituent are not included in the ring-forming atoms. The "number of ring atoms" described below is the same unless otherwise specified. For example, the number of ring atoms of a benzene ring is 6, the number of ring atoms of a naphthalene ring is 10, and the number of ring atoms of a thiophene ring is 5.

The "aryl" refers to hydrocarbyl containing at least one aromatic ring, including non-polycylic aryl and polycylic aryl. The polycylic aryl refers to a group formed after two or more aromatic rings are connected through two common adjacent ring atoms, that is, polycycle.

The "heteroaryl" refers to an aromatic ring group in which at least one ring-forming atom is a heteroatom. Heteroatoms include, but are not limited to, N, P, O, and S.

The "arylamino" refers to a group formed by a nitrogen atom being directly connected to one ring-forming atom in the above-mentioned aryl through a chemical bond.

Non-limiting examples of the "aryl" of the present invention include: benzene, naphthalene, anthracene, fluoranthene, phenanthrene, triphenylene, perylene, naphthacene, pyrene, benzopyrene, acenaphthene, or fluorene, etc.; non-limiting examples of "heteroaryl" include: triazine, pyridine, pyrimidine, imidazole, furan, thiophene, benzofuran, benzothiophene, indole, carbazole, pyrroloimidazole, pyrrolopyrrole, thienopyrrole, thienothiophene, furopyrrole, furofuran, thienofuran, benzisoxazole, benzisothiazole, benzimidazole, quinoline, isoquinoline, cinnoline, quinoxaline, phenanthridine, perimidine, quinazoline, quinazolinone, dibenzofuran, dibenzothiophene, carbazole, etc.; non-limiting examples of "arylamino" include: substituted or unsubstituted aniline, substituted or unsubstituted diphenylamine, or substituted or unsubstituted triphenylamine.

In the present invention, when a linking site is not specified in a group, it means that an optional linkable site in the group is used as the linking site.

In the present invention, a single bond connected to the substituent runs through the corresponding ring, indicating that the substituent is connectable to an optional position of the ring, for example, R in 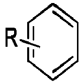 is connected to any substitutable site of a benzene ring, and when R substitutes H , this indicates that there is no substituent, and at this point, 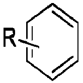 is benzene.

In the present invention, "substituted or unsubstituted" means that the defined group may be substituted or unsubstituted. When the defined group is substituted, it should be understood that it is optionally substituted by an acceptable group in the art, including but not limited to: C1-30 alkyl, heterocyclyl containing 3 to 20 ring atoms, aryl containing 5 to 20 ring atoms, and heteroaryl containing 5 to 20 ring atoms.

In the present invention, the same substituent may be independently selected from different groups at multiple occurrences. If 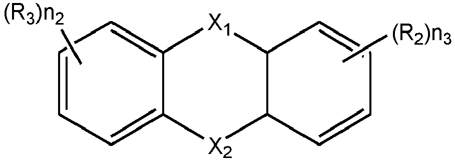 contains multiple R₃ or multiple R₂, each R₃ and R₂ may be independently selected from different groups. Further, when R₃ or R₂ is H, it means that a hydrogen on a benzene ring is not substituted by other substituents.

In the present invention, " 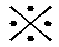 " represents a linking site.

In the present invention, when two groups are connected by a linking point, for example in 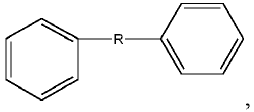 when R is selected from a single bond, it means that the two groups do not need to be connected through a specific group, but are directly connected by a single bond, that is

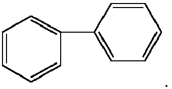

In the present invention, when two ring structures in the structural diagram share at least two ring-forming atoms, it means that the two ring structures are fused, and if a group of one of the ring structures is selected from H, it means that this ring structure does not exist. For example, in 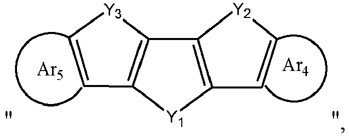 when Ar₄ or Ar₅ is selected from H, it means that Ar₄ or Ar₅ does not exist. If both Ar₄ and Ar₅ are H, this structure is

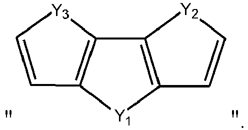

As mentioned in Background, when metal oxides such as nickel oxide are used as hole transport layer materials for the preparation of perovskite solar batteries, there will be excessive interface defects between a perovskite layer and a hole transport layer or hole transport speed will be unbalanced, increasing charge recombination at the interface, thereby reducing voltage at open circuit and fill factor of the solar battery, and in turn reducing photon-to-electron conversion efficiency and stability of the solar battery. Therefore, interface passivation is a main means used by researchers to improve efficiency of battery devices. A passivation layer is often added between the hole transport layer and the perovskite layer to passivate metal ions such as trivalent nickel.

In traditional technologies, passivation films are often prepared with polymers, for example, interface passivation films are prepared with polystyrene. However, skilled persons of the present application found during actual production and research and development that when passivation films are prepared with polymers such as polystyrene as passivation materials, it is difficult to control thickness and flatness, and if the thickness of the passivation films is too large, extraction of carriers would be inhibited, resulting in a significant decrease in battery efficiency. Moreover, the polymers are essentially mixtures of molecular chains with different molecular weights, molecules are different in degree of polymerization, which will lead to a large range of molecular weight changes, resulting in low repeatability of the passivation films. In addition, the polymers are not adapted to energy level matching of nickel oxide and perovskite, which will lead to an energy barrier during carrier extraction, making it more difficult for extraction, but resulting in a decrease in battery open circuit voltage.

One technology attempts to prepare a passivation layer by employing small molecular salts containing functional groups that may be combined with metals, passivating trivalent nickel using the functional groups that may be combined with metals. However, skilled persons of the present application accidentally found during actual production and research and development that it is also difficult for such small molecular salts to form a flat passivation film, so that improvements to light conversion efficiency and stability of the solar batteries prepared are very limited.

Thus, through a large number of creative experiments, skilled persons of the present application have obtained the organic compound in the present application that can passivate metals and have excellent film-forming properties. When used to prepare a passivation film, it can improve the photon-to-electron conversion efficiency and stability of solar battery devices.

An embodiment of the present application provides an organic compound, the organic compound being as shown in formula (1): 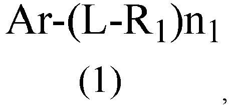 wherein Ar is selected from any one of aryl with 6 to 50 ring atoms, substituted or unsubstituted heteroaryl with 5 to 50 ring atoms, and substituted arylamino as shown in formula (A): 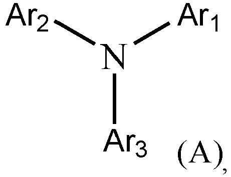
L is selected from acyclic alkylene with 1 to 10 carbon atoms; R₁ is an oxyacid group;
Ar₁ to Ar₃ are independently selected from any one of H, substituted or unsubstituted alkenyl with 2 to 30 carbon atoms, and substituted or unsubstituted aryl with 7 to 30 carbon atoms, respectively, and at least one of Ar₁ to Ar₃ is selected from substituted or unsubstituted aryl with 7 to 30 carbon atoms;
n₁ is selected from any integer from 1 to 3;
or the above-mentioned organic compound is an oxyacid radical salt of the compound as shown in formula (1).

In the above-mentioned organic compound, Ar, L, and R₁ with specific group structures are organically combined to form an organic compound of formula (1) or further form an oxyacid radical salt, wherein L is selected from acyclic alkylene with 1 to 10 carbon atoms and is connected with Ar and R₁ separately so as to form an organic whole, and R₁ is an oxyacid group, which may be combined with metal ions such as trivalent nickel so as to be able to play a role of passivating metal ions, while L plays a role of reducing steric hindrance. The organic compound formed by the organic combination of Ar, L and R₁ above may form an aggregate with an ordered structure through intermolecular interactions, has strong self-assembly ability so that a flat self-assembled passivation film can be prepared and the performance of the passivation film can be improved, and can improve photon-to-electron conversion efficiency and stability of a solar battery when used to prepare the solar battery.

Further, when the above-mentioned organic compound is applied to prepare a passivation film for a solar battery, on the one hand, regulating the number of carbon atoms in L may improve hydrophobicity of the passivation film, so as to further inhibit the negative effects of water and oxygen on perovskite light-absorbing material; on the other hand, the specific group structure in Ar enables the organic compound to have an energy level adapted to other functional layer materials in the solar battery, so that the photoconversion efficiency and stability of the solar battery may be further improved.

Moreover, in the organic compound of the present application, mutual attraction between Ar and R₁ with specific structural groups is small. Thus, in the process of self-assembly film formation of the organic compound, the mutual attraction of molecular head and tail structures of the organic compound may be avoided, ensuring molecules of the organic compound form aggregates with an ordered structure through intermolecular interactions, so that film formation proceeds in a direction of forming a flat single-layer film, which is conducive to the formation of a passivation film with uniform thickness, thereby improving light conversion efficiency and stability of solar batteries.

In some of the Examples, acyclic alkylene is linear alkylene.

Preferably, L is selected from acyclic alkylene with 3 to 11 carbon atoms.

Further regulating the number of carbon atoms in L improves hydrophobicity of the organic compound while reducing steric hindrance of the organic compound, this further improves the conversion efficiency and stability of a solar battery.

In some of the Examples, L is selected from linear alkylene with 3 to 11 carbon atoms.

Further, L is selected from linear alkylene with 3 to 8 carbon atoms.

Still further, L is selected from linear alkylene with 3 to 6 carbon atoms.

Non-limiting examples of L include the following structures: 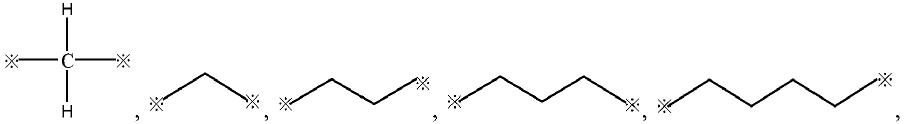 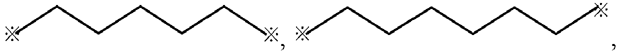 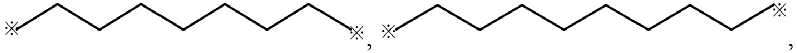 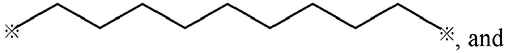 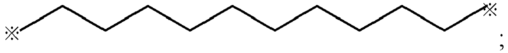 wherein 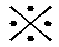 represents a linking site.

In some of the Examples, R₁ is, at each occurrence, independently selected from any one of a phosphonic acid group, a sulfonic acid group, a carboxylic acid group, a sulfinic acid group, a boric acid group, and a silicic acid group, respectively.

R₁ is, at each occurrence, independently selected from any one of the following structures, respectively: 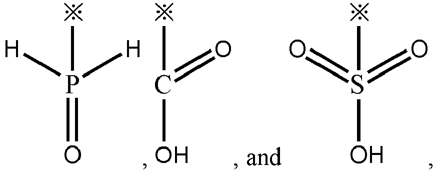 and 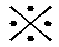 represents a linking site.

In some of the Examples, heteroatoms in the above-mentioned substituted or unsubstituted heteroaryl with 5 to 50 ring atoms are selected from at least one of N, O, and S.

When Ar is substituted or unsubstituted heteroaryl with 5 to 50 ring atoms, the heteroatoms are selected from at least one of N, O, and S, so that the organic compound has an energy level more adapted to common metal oxide hole transport materials and perovskite material, and when applied to the preparation of a passivation film for a solar battery, further improves performance of the solar battery.

In some of the Examples, at least one heteroatom in the above-mentioned substituted or unsubstituted heteroaryl with 5 to 50 ring atoms is N.

In some of the Examples, Ar is selected from any one of aryl with 6 to 30 ring atoms, heteroaryl with 5 to 30 ring atoms, or arylamino as shown in formula (A).

In some of the Examples, Ar is selected from any one of fused aryl with 8 to 30 ring atoms, fused heteroaryl with 8 to 30 ring atoms, or arylamino substituted or as shown in formula (A).

Ar is selected from any one of formulas (A) to (C): 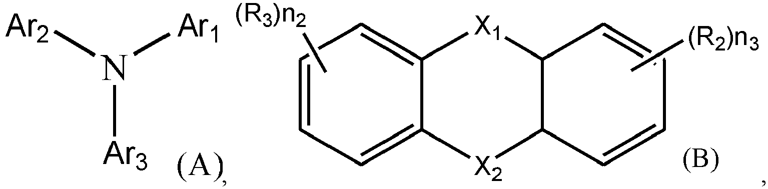 and 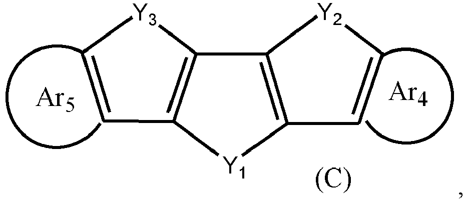 and 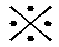 represents a linking site. X₁ and X₂ are independently selected from any one of a single bond, C(R₄R₅), O, S, N, NR₄, C=O, or S=O, respectively, and X₁ and X₂ are not single bonds at the same time.

It should be noted that, in the above formula, X₁ and X₂ are not single bonds at the same time, and N exists in two forms in this formula: N and NR₄, for example, when forming a pentacyclic ring, it may be

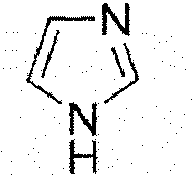

In some of the Examples, X₁ and X₂ are independently selected from any one of a single bond, C(R₄R₅), O, S, NR₄, C=O, or S=O, respectively, and X₁ and X₂ are not single bonds at the same time.

In some of the Examples, X₁ and X₂ are independently selected from any one of a single bond, C(R₄R₅), NR₄, C=O, or S=O, respectively, and X₁ and X₂ are not single bonds at the same time.

In some of the Examples, X₁ and X₂ are independently selected from any one of a single bond, C(R₄R₅) and NR₄, respectively, and X₁ and X₂ are not single bonds at the same time.

Y₁, Y₂, and Y₃ are independently selected from any one of C(R₄R₅), O, S, N, NR₄, C=O, or S=O, respectively.

In some of the Examples, Y₁, Y₂, and Y₃ are independently selected from any one of C(R₄R₅), O, S, N, and NR₄, respectively.

In some of the Examples, Y₁, Y₂, and Y₃ are independently selected from any one of C(R₄R₅), O, S, and NR₄, respectively.

In some of the Examples, R₄ in the above-mentioned NR₄ is, at each occurrence, independently selected from any one of H, D, substituted or unsubstituted alkyl with 1 to 5 carbon atoms, and the following structures, respectively: 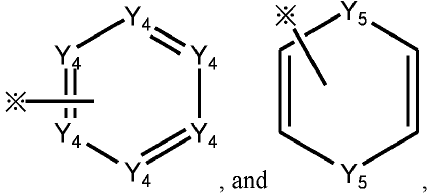
Y₄ is, at each occurrence, independently selected from CR₉ or N, respectively, and Y₄ in the same structural formula is not N at the same time;
Y₅ is, at each occurrence, separately and independently selected from CR₉R₁₀ or NR₉;
R₉ to R₁₀ are, at each occurrence, independently selected from any one of H, D, substituted or unsubstituted linear alkyl with 1 to 10 carbon atoms, substituted or unsubstituted branched alkyl with 3 to 20 carbon atoms and substituted or unsubstituted cycloalkyl with 3 to 20 carbon atoms, respectively. 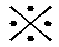
represents a linking site with N in NR₄.
R₂ to R₅ are, at each occurrence, independently selected from any one of H, D, a halogen group, -N(R₆)₂, -CONR₆, -OCOR₆, substituted or unsubstituted alkyl with 1 to 30 carbon atoms, substituted or unsubstituted aryl with 6 to 30 carbon atoms, and substituted or unsubstituted heteroaryl with 5 to 30 carbon atoms, respectively.
R₂ to R₅ are, at each occurrence, independently selected from any one of H, D, a halogen group, -N(R₆)₂, -CONR₆, -OCOR₆, substituted or unsubstituted linear alkyl with 1 to 20 carbon atoms, substituted or unsubstituted aryl with 6 to 20 carbon atoms, and substituted or unsubstituted heteroaryl with 5 to 20 carbon atoms, respectively.

The halogen group includes any one of F, Cl, Br, and I.

The above-mentioned -CONR₆ represents an amide compound, with a structure that may be expressed as -C(=O)NR₆; -OCOR₆ represents an ester compound, with a structure that may be expressed as -OC(=O)R₆.

R₆ is, at each occurrence, independently selected from any one of H, D, substituted or unsubstituted alkyl with 1 to 30 carbon atoms, substituted or unsubstituted alkenyl with 2 to 30 carbon atoms, substituted or unsubstituted aryl with 6 to 30 carbon atoms, and substituted or unsubstituted heteroaryl with 5 to 30 carbon atoms, respectively.

In some of the Examples, R₆ is, at each occurrence, independently selected from any one of H, D, unsubstituted linear alkyl with 1 to 15 carbon atoms, unsubstituted linear alkenyl with 2 to 10 carbon atoms, unsubstituted aryl with 6 to 20 carbon atoms, and unsubstituted heteroaryl with 5 to 20 carbon atoms, respectively.

Ar₄ and Ar₅ are independently selected from any one of H, substituted or unsubstituted aryl group with 6 to 30 carbon atoms, and substituted or unsubstituted heteroaryl with 5 to 30 carbon atoms, respectively.

In some of the Examples, Ar₄ and Ar₅ are independently selected from any one of H, aryl with 6 to 20 carbon atoms, and heteroaryl with 5 to 20 carbon atoms, respectively.

n₂ and n₃ are independently selected from any integer from 1 to 4, respectively.

n₂ and n₃ are independently selected from 1, 2, 3, or 4, respectively.

In some of the Examples, Ar₄ and Ar₅ are independently selected from any one of H and the following structures, respectively: 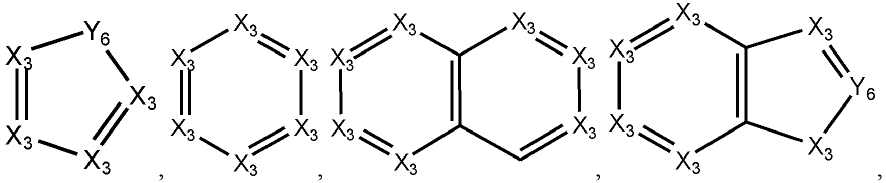 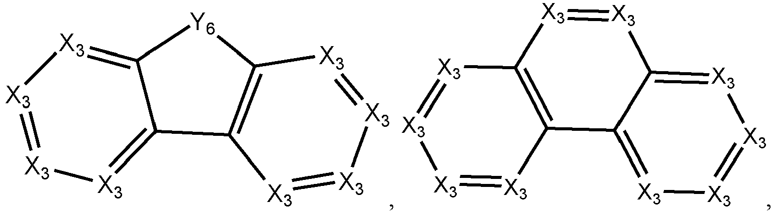 and 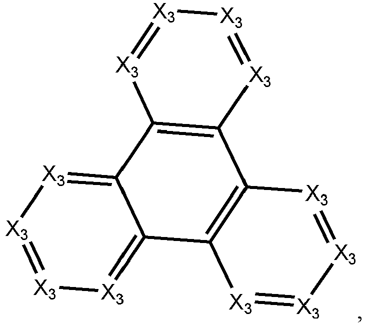 where: Y₆ is selected from any one of CR₇R₈, O, S, S=O, or C=O.

In some of the Examples, Y₆ is selected from any one of CR₇R₈, O, and S.

X₃, at each occurrence, separately and independently represents CR₇ or N, and X₃ in the same structural formula is not N at the same time.

R₇ and Rs are, at each occurrence, independently selected from any one of H, D, substituted or unsubstituted linear alkyl with 1 to 10 carbon atoms, substituted or unsubstituted branched alkyl with 3 to 20 carbon atoms and substituted or unsubstituted cycloalkyl with 3 to 20 carbon atoms, respectively.

In some of the Examples, Ar₁ to Ar₃ are independently selected from any one of H, alkenyl with 2 to 30 carbon atoms, and aryl with 7 to 30 carbon atoms, respectively, and at least one of Ar₁ to Ar₃ is selected from aryl with 7 to 30 carbon atoms.

In some of the Examples, Ar₃ to Ar₅ are independently selected from any one of H, cycloalkenyl with 6 to 10 carbon atoms, and aryl with 7 to 20 carbon atoms, respectively, and at least one of Ar₃ to Ar₅ is selected from aryl with 7 to 20 carbon atoms.

In some of the Examples, at least one of Ar₁ to Ar₃ is selected from any one of following structures,

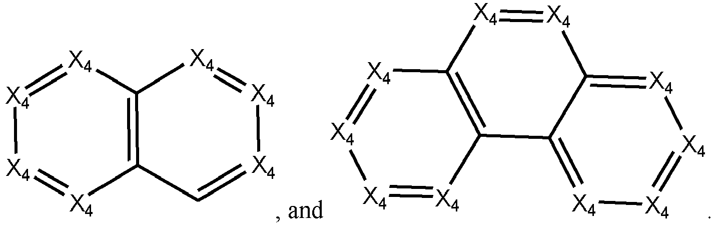

X₄, at each occurrence, separately and independently represents CR₁₁.

R₁₁ is, at each occurrence, independently selected from H, D, substituted or unsubstituted linear alkyl with 1 to 10 carbon atoms, substituted or unsubstituted branched alkyl with 3 to 20 carbon atoms and substituted or unsubstituted cycloalkyl with 3 to 20 carbon atoms, respectively.

In some of the Examples, Ar is selected from any one of following structures: 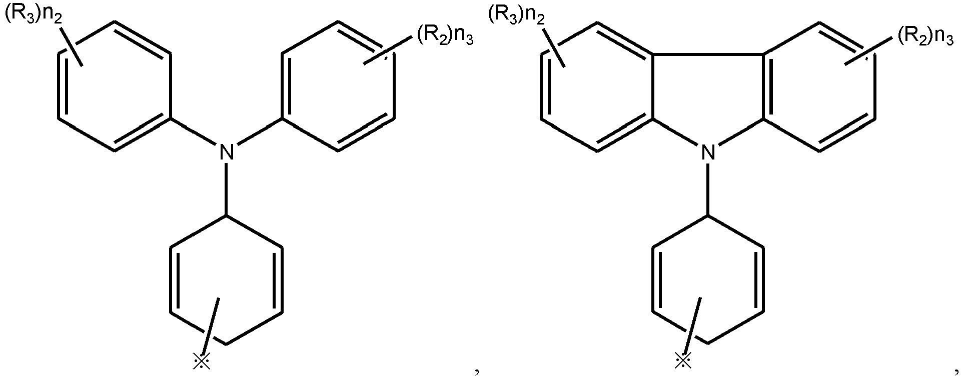 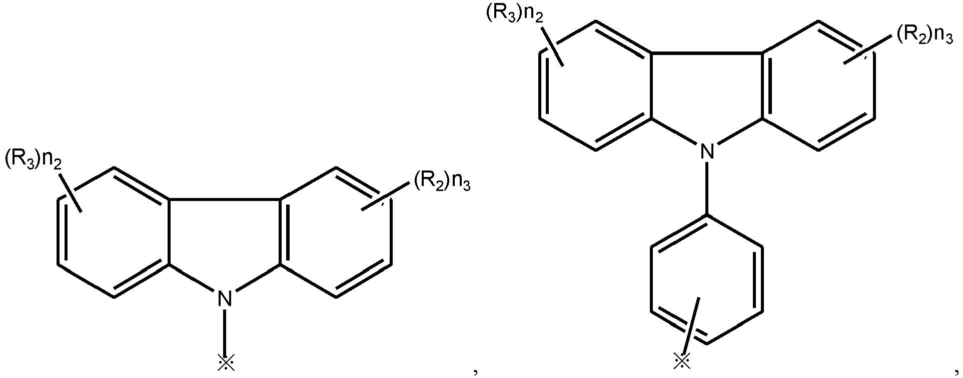 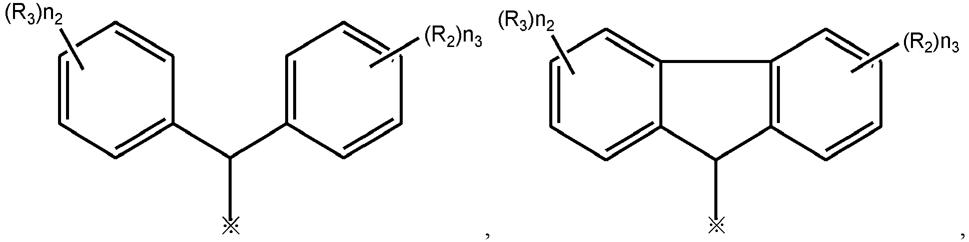 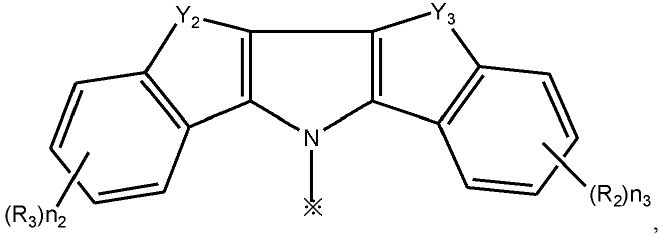 and

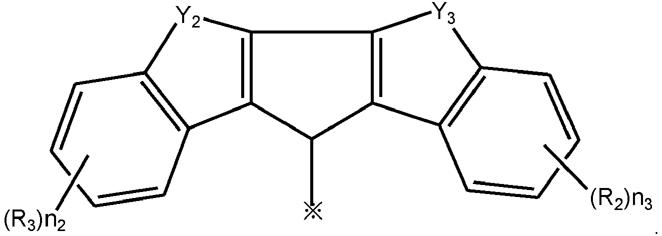

The meanings of R₂ to R₃ and n₂ to n₃ are the same as above.

In some of the Examples, the oxyacid radical salt of the compound as shown in formula (1) above includes an anion and a cation, the anion is formed by at least one alcoholic hydroxyl in an oxyacid group in the compound as shown in formula (1) above losing H, and the above-mentioned cation is selected from a metal ion or NH₄⁺.

In some of the Examples, the cation is selected from any one of Na⁺, K⁺, Mg²⁺, Li⁺, Ca²⁺, Cu²⁺, Fe²⁺, Mn²⁺, Zn²⁺, Sn²⁺, or NH₄⁺.

It should be noted that when valence state of the above-mentioned cation is +2 valence and higher, the valence state of the corresponding anion is a respective negative valence state, and if the valence state of the corresponding anion is +1 valence, then the valence state of the anion is two or more. In other words, in an oxyacid radical salt of the compound as shown in formula (1) above, an algebraic sum of the valence states of the oxyacid radical salt and cation of the compound as shown in formula (1) above is 0.

An embodiment of the present invention provides use of an organic compound as a metal deactivator , the organic compound being as shown in formula (1): 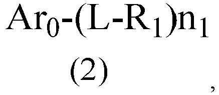
wherein Arc is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group;
n₁ is selected from any integer from 1 to 3;
or the organic compound is an oxyacid radical salt of the compound as shown in formula (2).

The meanings and selection ranges of L, R₁ and n₁ are the same as above.

In some of the Examples, in Ar₀, heteroatoms in the above-mentioned substituted or unsubstituted heteroaryl with 5 to 50 ring atoms are selected from at least one of N, O, and S.

When Arc is substituted or unsubstituted heteroaryl with 5 to 50 ring atoms, the heteroatoms are selected from at least one of N, O, and S, so that the organic compound has an energy level more adapted to common metal oxide hole transport materials and perovskite material, and when applied to the preparation of a passivation film for a solar battery, further improves performance of the solar battery.

In some of the Examples, in Ar₀, at least one heteroatom in the above-mentioned substituted or unsubstituted heteroaryl with 5 to 50 ring atoms is N.

In some of the Examples, Arc is selected from any one of substituted or unsubstituted aryl with 6 to 30 ring atoms, substituted or unsubstituted heteroaryl with 5 to 30 ring atoms, and substituted or unsubstituted arylamino with 6 to 30 ring atoms.

In some of the Examples, Ar is selected from any one of substituted or unsubstituted fused aryl with 8 to 30 ring atoms, substituted or unsubstituted fused heteroaryl with 8 to 30 ring atoms, and substituted or unsubstituted arylamino with 6 to 30 ring atoms.

Ar is selected from any one of formulas (B) to (D): 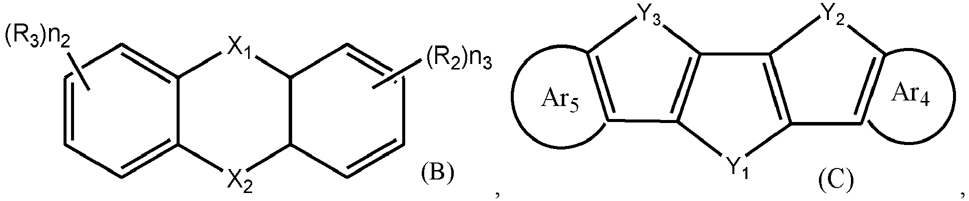 and 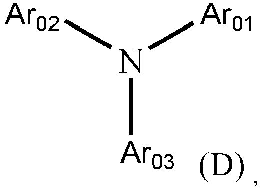 and 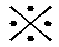 represents a linking site.

X₁, X₂, Y₁, Y₂, Y₃, Y₄, Y₅, R₂ to R₅, Ar₄ and Ar₅, as well as n₂ and n₃ have the same meanings and selection ranges as above.

In some of the Examples, Ar₀₁ to Ar₀₃ are independently selected from any one of H, substituted or unsubstituted alkenyl with 2 to 30 carbon atoms, and substituted or unsubstituted aryl with 6 to 30 carbon atoms, respectively, and at least one of Ar₀₁ to Ar₀₃ is selected from substituted or unsubstituted aryl with 6 to 30 carbon atoms.

In some of the Examples, Ar₀₁ to Ar₀₃ are independently selected from any one of H, cycloalkenyl with 6 to 10 carbon atoms, and aryl with 6 to 20 carbon atoms, respectively, and at least one of Ar₀₁ to Ar₀₃ is selected from aryl with 6 to 20 carbon atoms.

In some of the Examples, at least one of Ar₀₁ to Ar₀₃ is selected from any one of following structures,

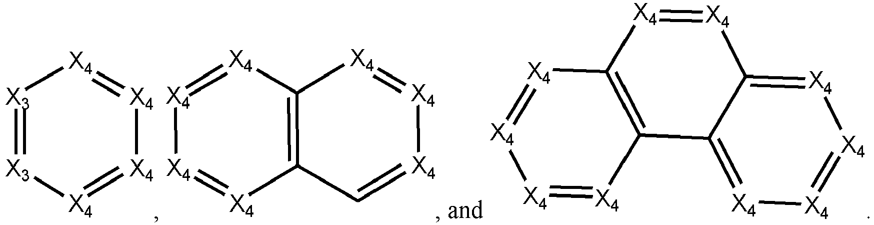

The meaning and selection range of X₄ are the same as above.

In some of the Examples, Arc is selected from any one of following structures: 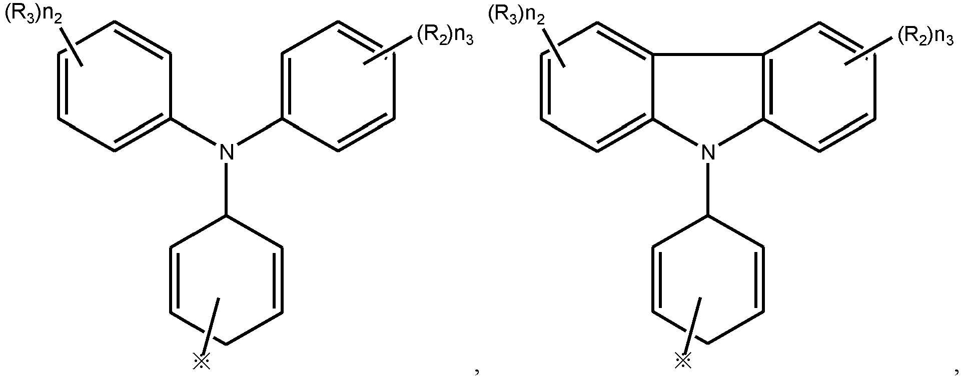 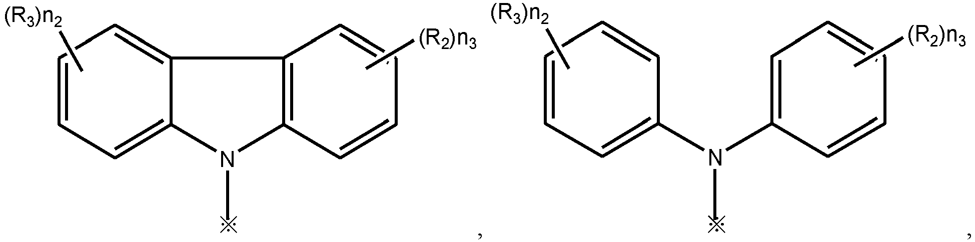 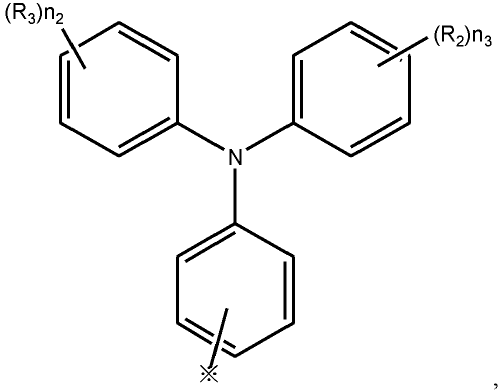 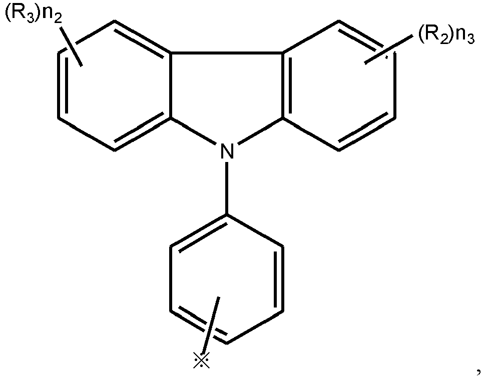 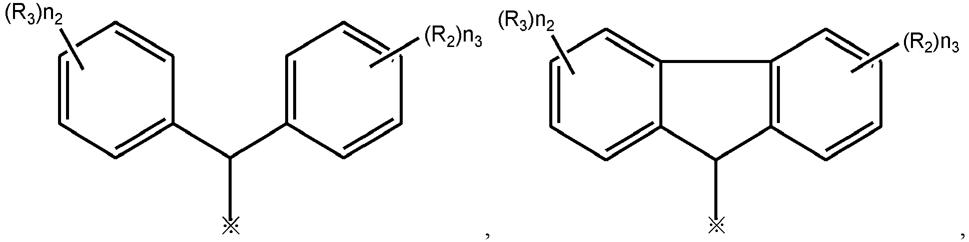 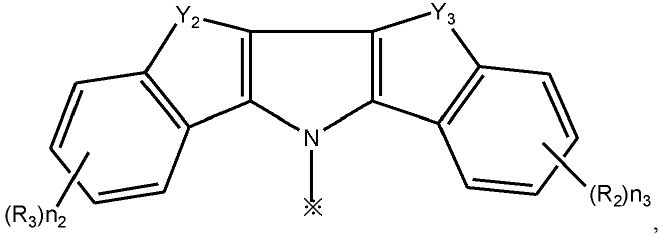 and

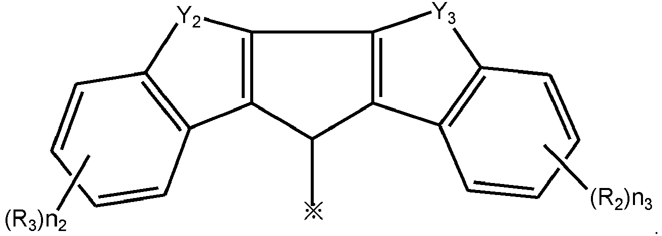

The meanings of R₂ to R₃ and n₂ to n₃ are the same as above.

In the technical solutions of the Examples of the present application, Ar₀, L, and R₁ with specific group structures are organically combined to form the organic compound of formula (1) or further form an oxyacid radical salt, wherein L is selected from acyclic alkylene with 1 to 10 carbon atoms and is connected with Arc and R₁ separately so as to form an organic whole, and R₁ is an oxyacid group, which may be combined with metal ions such as trivalent nickel so as to play a role of passivating metal ions.

In some of the Examples, the above-mentioned metal deactivator is a nickel metal deactivator, a zinc metal deactivator, or a molybdenum metal deactivator.

An embodiment of the present invention provides use of an organic compound in the preparation of a passivation film. The organic compound is as shown in formula (2), or the organic compound is an oxyacid radical salt of the compound as shown in formula (2).

The structure of formula (2) or the oxyacid radical salt of the compound as shown in formula (2) is the same as above, and will not be repeated.

An embodiment of the present invention provides a passivation film, components of which include the organic compound as shown in formula (2) and/or the oxyacid radical salt of the compound as shown in formula (2).

The structure of formula (2) or the oxyacid radical salt of the compound as shown in formula (2) is the same as above, and will not be repeated.

In the above-mentioned organic compound, L is selected from acyclic alkylene with 1 to 10 carbon atoms and is connected with Arc and R₁ separately so as to form an organic whole, and R₁ is an oxyacid group, which may be combined with metal ions such as trivalent nickel so as to be able to play a role of passivating metal ions, while L plays a role of reducing steric hindrance. The organic compound formed by the organic combination of the above-mentioned Arc, L and R₁ may form an aggregate with an ordered structure through intermolecular interactions, has strong self-assembly ability so that a flat self-assembled passivation film can be prepared and the performance of the passivation film can be improved. Further, on the one hand, regulating the number of carbon atoms in L may improve hydrophobicity of the passivation film, so as to further inhibit the negative effects of water and oxygen on perovskite light-absorbing material; on the other hand, the specific group structure in Arc enables the organic compound to have an energy level adapted to other functional layer materials in a solar battery, so that the photoconversion efficiency and stability of the solar battery may be further improved.

In some of the Examples, the above-mentioned passivation film is a single-layer film.

In some of the Examples, the above-mentioned passivation film is 0.1 nm to 10 nm in thickness.

In some of the Examples, the above-mentioned passivation film is 0.1 nm to 5 nm in thickness.

An embodiment of the present invention also provides a method for preparing the above-mentioned passivation film, including the following steps S10 to S20.

At step S10, the organic compound as shown in formula (2) and/or the oxyacid radical salt of the compound as shown in formula (2) and a solvent are mixed to prepare a mixed solution.

The structure of formula (2) or the oxyacid radical salt of the compound as shown in formula (2) is the same as above, and will not be repeated.

At step S20, a surface of a substrate is coated with the mixed solution and dried to obtain a passivation film.

In some of the Examples, the above-mentioned solvent is an organic solvent; further, the above-mentioned solvent is selected from at least one of alcohol solvents, aromatic solvents, amide solvents, and sulfoxide solvents.

In some of the Examples, the above-mentioned solvent is selected from at least one of methanol, isopropanol, ethanol, and chlorobenzene.

In some of the Examples,the mixed solution is 0.1 mg/mL to 10 mg/mL in concentration.

By adjusting the concentration of the mixed solution, the thickness of the passivation film prepared may be further adjusted.

In some of the Examples, the above-mentioned coating step may adopt spin coating, blade coating, slot coating and the like.

In some of the Examples, the above-mentioned drying step is used to remove the solvent, and the solvent may be removed by means of annealing or vacuum heating.

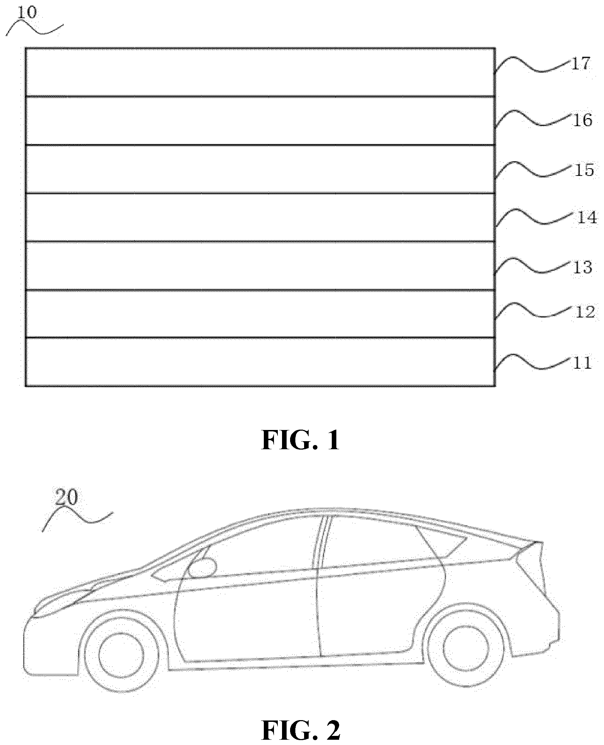

Referring to FIG. 1, the present application provides a solar battery 10, the solar battery 10 includes a hole transport layer 12 and a passivation layer 13 that are stacked, components of the hole transport layer 12 include a metal oxide, and the passivation layer 12 includes the organic compound as shown in formula (2) and/or the oxyacid radical salt of the compound as shown in formula (2).

The structure of formula (2) or the oxyacid radical salt of the compound as shown in formula (2) is the same as above.

In the organic compound in the passivation layer 12, L is selected from acyclic alkylene with 1 to 10 carbon atoms and is connected with Arc and R₁ separately so as to form an organic whole. R₁ is an oxyacid group, which may be combined with metal ions such as trivalent nickel so as to be able to play a role of passivating metal ions. A specific group structure in Ar₀ enables the organic compound to have an energy level adapted to the hole transport layer 12. The organic compound formed by the organic combination of the above-mentioned Arc, L, and R₁ may form an aggregate with an ordered structure through intermolecular interactions, thereby enabling photon-to-electron conversion efficiency and stability of a solar battery to be improved.

It may be understood that the metal oxides in the above-mentioned hole transport layer 12 may be various metal oxides commonly used in the field that may be used as hole transport materials.

In some of the Examples, components of the hole transport layer 12 include at least one of nickel oxide, zinc oxide, and molybdenum oxide.

With continued reference to FIG. 1, the above-mentioned solar battery 10 further includes a perovskite layer 14, and the perovskite layer 14 is located on a surface of the passivation layer 13 away from the hole transport layer 12.

The organic compound in the passivation layer 12 may passivate the metal ions such as trivalent nickel in the hole transport layer 12, thereby reducing grain boundary defects occurring in the perovskite layer 14, and the organic compound in the passivation layer 12 has an energy level adapted to perovskite material and excellent hydrophobicity, can inhibit the negative impact of water and oxygen on perovskite light-absorbing material, and can, in turn, improve the photon-to-electron conversion efficiency and stability of the solar battery.

It may be understood that components in the perovskite layer 14 include perovskite material commonly used in the art.

In some of the Examples, a chemical formula of the perovskite material satisfies ABX₃ or A₂CDX₆; wherein A is an inorganic cation or an organic ammonium cation or a mixture of both, which may be at least one of formamidine ion (FA), methylammonium ion (MA), and Cs; B is an inorganic metal cation, which may be at least one of Pb ion and Sn ion; C is a noble metal cation, usually Ag+; D is a heavy metal or rare metal cation, which may be at least one of bismuth cation Bi³⁺, antimony cation Sb³⁺, and indium cation In³⁺; X oxygen or a halogen element may be at least one of O, Br, and I.

In some of the Examples, the above-mentioned perovskite layer 14 has a bandgap of 1.20eV to 2.30eV, and a thickness of 200 nm to 1000 nm.

With continued reference to FIG. 1, the solar battery 10 further includes a first electrode 11, an electron transport layer 15, and a second electrode 17.

The first electrode 11 is located on a surface of the hole transport layer 12 away from the passivation layer 13, the electron transport layer 15 is located on a surface of the perovskite layer 14 away from the passivation layer 13, and the second electrode 17 is located on a surface of the electron transport layer 15 away from the perovskite layer 14.

In some of the Examples, the first electrode 11 is a transparent conductive electrode, and the first electrode 11 may be made from FTO, ITO, AZO, BZO, and IZO.

In some of the Examples, components in the electron transport layer 15 may be electron transport materials commonly used in the art, and non-limiting examples include: methyl [6,6]-phenyl-C61-butyrate (PC61BM), methyl [6,6]-phenyl-C71-butyrate (PC71BM), fullerene C60 (C60), fullerene C70 (C70), tin dioxide (SnO2), zinc oxide (ZnO), etc.

In some of the Examples, the second electrode 17 may be made from electrode materials commonly used in the art, including but not limited to the following materials: Ag, Cu, C, Au, Al, ITO, AZO, BZO, IZO, etc.

In some of the Examples, with continued reference to FIG. 1, the solar battery 10 further includes a buffer layer 16, and the buffer layer 16 is located between the electron transport layer 15 and the second electrode 17.

Components of the buffer layer 16 may be the organic compounds mentioned above, or other buffer passivating materials in the art.

An embodiment of the present invention further provides an electrical apparatus, and the electrical apparatus includes the above-mentioned solar battery.

The above-mentioned solar battery has high light conversion efficiency and good stability, and may improve the efficiency and extend service life of the electrical apparatus.

In some of the Examples, the above-mentioned solar battery may serve as a power source for the electrical apparatus, and may also serve as an energy storage unit for the electrical apparatus.

Further, the above-mentioned electrical apparatus may include a mobile device (e.g., a mobile phone, a laptop, etc.), an electric vehicle, an electric train, ship, satellite, energy storage system and the like, but is not limited thereto.

FIG. 2 shows an electrical apparatus as an example. The electrical apparatus 20 is an all-electric vehicle, a hybrid electric vehicle, a plug-in hybrid electric vehicle, or the like. the electrical apparatus as another example may be a mobile phone, a tablet, a laptop, etc.

### Specific Examples

### Example 1

### (1) A following compound A was provided:

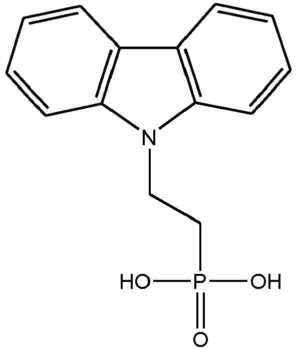

Specific steps are as follows:

### (1) Synthesis of 9-(2-bromoethyl)-9H-carbazole:

1.672 g of carbazole was dissolved in 25 mL of 1,2-dibromoethane, and mixed evenly by stirring. 0.5 g of tetrabutylammonium bromide and 3 mL of 50wt% KOH aqueous solution were then added. The temperature was raised to 50°C. After stirring for 72 h, reaction was stopped. A product was extracted with dichloro. An organic phase was dried with anhydrous MgSO₄, and a crude product was obtained by rotary evaporation to remove the solvent. The crude product was purified through a column. A mobile phase was a mix of acetone and n-hexane with a volume ratio of 1:10.

### (2) Synthesis of diethyl 2-(9H-carbazole-9-ethyl) phosphonate:

1 g of 9-(2-bromoethyl)-9H-carbazole was dissolved in 15 mL of triethyl phosphite, subjected to heating reflux, and reacted for 24 h. After reaction, most of the organic solvent was removed by rotary evaporation. A product was purified through a column. A mobile phase was a mixed solution of acetone and n-hexane with a volume ratio of 1:4. Diethyl 2-(9H-carbazole-9-ethyl) phosphonate was obtained, with a structure shown as below:

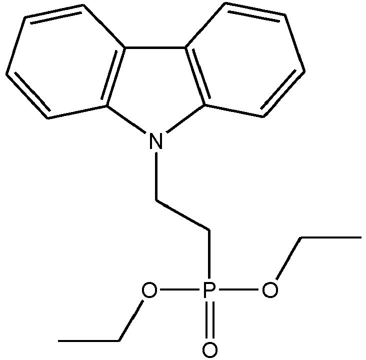

(3) Synthesis of 2-(9H-carbazole-9-ethyl) phosphonic acid (Compound A): 1g of diethyl 2-(9H-carbazole-9-ethyl) phosphonate was taken and dissolved in 20 mL ethanol. 10 mL NaOH saturated solution was added, and reacted at 60°C for 24 h, and hydrochloric acid was then dropwise added to adjust pH to acidic state. The solvent was removed by rotary evaporation after reaction. The resultant was dissolved in dichloromethane, washed with saturated brine, and then dried with anhydrous magnesium sulfate. Most of the solvent was removed by concentrating to obtain a crude product. The crude product was purified through a column. A mobile phase was a mixed solution of ethyl acetate and n-hexane with a volume ratio of 1:4.

The obtained target product compound A was subjected to a nuclear magnetic resonance test, and data were as follows:
1H NMR: δ 1.83 (2H, s), δ 2.83 (2H, t, J = 6.1 Hz), 4.24 (2H, t, J = 6.1 Hz), 7.13 (2H, ddd, J = 8.0, 7.6, 2.3 Hz), 7.30 (2H, ddd, J = 8.0, 7.6, 1.6 Hz), 7.52 (2H, ddt, J = 8.0, 2.3, 0.5 Hz), and 7.68 (2H, ddd, J = 8.0, 1.6, 0.5 Hz).

### (2) Preparation of solar battery. Specific steps are as follows:

1. 20 Pieces of FTO conductive glass with a size of 2.0 * 2.0 cm were taken, and 0.35 cm of FTO at both ends of each piece was removed by laser etching to expose a glass substrate.
2. The etched FTO conductive glass was ultrasonically washed with water, acetone, and isopropanol in sequence.
3. The cleaned FTO conductive glass was blown under a nitrogen gun to dry a solvent, and was put into an ultraviolet ozone machine for ultraviolet ozone cleaning.
4. After 10 mg/mL nickel oxide particleboards (water as a solvent) were applied on the FTO substrate by spin coating at a rate of 4000 rpm/s after ultraviolet ozone cleaning to obtain a hole transport layer, then annealing was performed on a hot stage at 100°C for 30 minutes.
5. The above-mentioned compound A was dissolved in methanol to obtain a self-assembled molecule solution with a concentration of 0.5 mg/mL after dissolution; self-assembled molecules are applied on the nickel oxide substrate at 3000 rpm/s, and drying was performed in a vacuum manner to form a passivation layer with a thickness of 1 nm.
6. 223 mg of lead iodide PbI₂, 80 mg of formamidine iodide FAI, and 15 mg of methylamine chloride (MACl) were weighed and dissolved in a mixed solution of 0.8 mL DMF and 0.2 mL DMSO. Stirring was performed for 3 h. Filtering was performed with a 0.22 µm organic filter membrane to obtain a perovskite precursor solution. The perovskite precursor solution was applied on the passivation layer by spin coating at 3000 rpm/s. Annealing was performed at 100°C for 30 min before cooling to room temperature, and a perovskite layer was formed, in which an active material in the perovskite layer was CsFAMA system and has a thickness of 500nm.
7. PCBM was dissolved in chlorobenzene to form an electron transport material solution with a concentration of 20 mg/mL. The electron transport material solution was applied on the perovskite layer by spin coating at 1500 rpm/s before annealing at 100°C for 10 min to form an electron transport layer with a thickness of 50 nm; a passivation material BCP solution (BCP was dissolved in isopropanol, and a concentration was 0.5 mg/mL) was then immediately applied by spin coating at 5000 rpm/s to form a passivation layer with a thickness of 5 nm. The resultant was then put into an evaporation machine for evaporating a metal electrode Ag, and an obtained battery device was marked as battery 1.

### (3) Performance of the solar battery prepared was tested. Specific steps are as follows:

Testing was conducted in accordance with the national standard IEC61215 using a solar simulator from Enlitech. Intensity of light was calibrated using a crystalline silicon solar battery so that it reached a solar intensity, AM1.5. A battery was connected to a digital source meter, and its photon-to-electron conversion efficiency was measured under light. The specific results are shown in Table 1.

### Example 2

Example 2 was the same as Example 1, the only difference was that compound B (CAS: 13505-00-5) was used in a fifth stage of step (2) to prepare a passivation layer, and the structure of the compound was shown as follows:

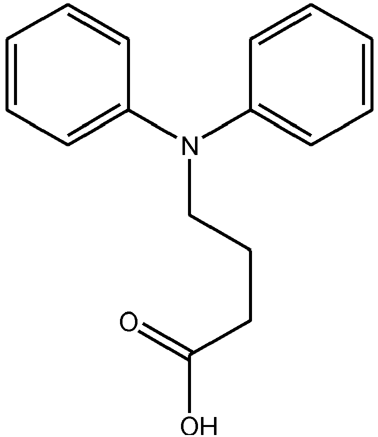

Other steps and conditions are the same as those in Example 1.

### Example 3

Example 3 was the same as Example 1, the only difference was that compound C was used in a fifth stage of step (2) to prepare a passivation layer, and the structure of the compound was shown as follows:

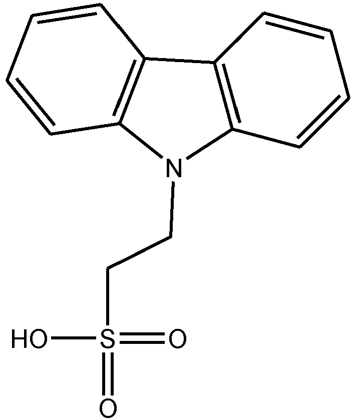

### Specific steps are as follows:

(1) In a round-bottom flask 1, 3.26 g (20 mmol) of 2-chloroethanesulfonyl chloride, 1.2 g (20 mmol) of isopropanol, and 40 ml of 1,2-dichloroethane were mixed, and the temperature was lowered to 0°C. Another flask was taken. 3.34 g (20 mmol) of carbazole was weighed and dissolved in 20 ml of 1,2-dichloroethane, and stirred sufficiently until full dissolution to obtain a carbazole solution. The carbazole solution was added dropwise into the round-bottom flask 1. After the dropwise addition, the reaction temperature was returned to room temperature, and the reaction was continued for 4 h. After suction filtration, drying, and concentration, a crude product was purified through a column, and a mobile phase was a mixed solution of dichloromethane and n-hexane with a volume ratio of 1:8. An intermediate C-1 was obtained by purifying, with a structure shown as follows: 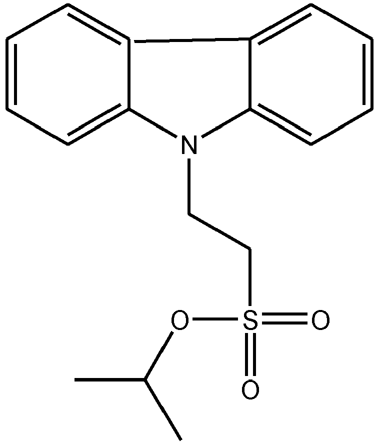
(2) In a round-bottom flask that was taken, the intermediate C-1 was dissolved in ethanol. A vial was additionally taken to prepare a saturated sodium hydroxide solution. Sodium hydroxide was added dropwise into product 1 until the solution became cloudy and pH value was 11. The temperature was adjusted to 60°C. Stirring was continued for hydrolysis reaction for 3 h. Hydrochloric acid was then added dropwise to adjust the pH value. When pH was adjusted to 2, the dropwise addition was stopped. The ethanol in the system was removed by rotary evaporation. A small amount of water was added, and extracting was conducted with ethyl acetate. Oil phases were combined, dried, and concentrated. A crude product was purified through a column. A mobile phase was a mixed solution of ethyl acetate and n-hexane with a volume ratio of 1:4. Compound C was obtained by purifying.

The obtained target product C was subjected to a nuclear magnetic resonance test, and data were as follows:
1H NMR: δ 2.53 (1H, s), δ 3.79 (2H, t, J = 6.5 Hz), 4.29 (2H, t, J = 6.5 Hz), 7.13 (2H, ddd, J = 8.0, 7.6, 2.3 Hz), 7.30 (2H, ddd, J = 8.0, 7.6, 1.6 Hz), 7.53 (2H, ddt, J = 8.0, 2.3, 0.5 Hz), and 7.69 (2H, ddd, J = 8.0, 1.6, 0.5 Hz).

Other steps and conditions are the same as those in Example 1.

### Example 4

Example 4 was the same as Example 1, the only difference was that compound D was used in a fifth stage of step (2) to prepare a passivation layer, and the structure of the compound was shown as follows:

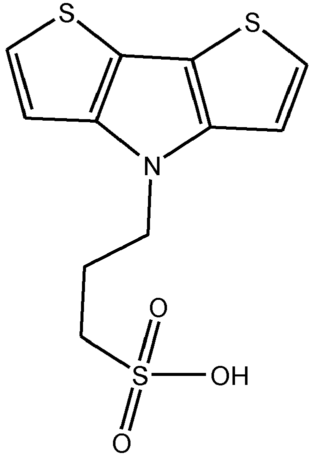

### Specific steps are as follows:

(1) In a clean and dry reaction flask that was a round-bottom flask, and 7 g 3,3'-dibromo-2,2'-bithiophene (21.60 mmol), 1.23 g CuI (21.60 mmol), 2.29 g dimethylethylenediamine (25.92 mmol), and 10.45 g potassium carbonate (75.61 mmol) were mixed. 70 mL distilled toluene and 0.1 mL of deionized water were then added. An atmosphere in the reaction flask was flushed and replaced with nitrogen, and 3.14 g benzamide (25.92 mmol) was then added. After the addition, argon was used for filling and venting three times, sealing was performed, and an argon balloon was inserted. The reaction was refluxed at 110°C for 48h, and then cooled to room temperature. Then, a crude product was purified through column chromatography (silica gel, a mobile phase was a mixed solution of dichloromethane and petroleum ether with a volume ratio of 1:2) to obtain white crystalline solid 4H-dithienopyrrole, with a structure shown as follows: 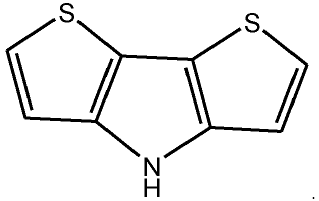
(2) 3.58 g (20 mmol) of 3-chloropropanesulfonyl chloride, 1.2 g (20 mmol) of isopropanol, and 40 ml of 1,2-dichloroethane were mixed in a round-bottom flask 1, and the temperature was lowered to 0°C. Another flask was taken. The 4H-dithienopyrrole (20 mmol) was weighed and dissolved in 20 ml of 1,2-dichloroethane, and stirred sufficiently until full dissolution to obtain a 4H-dithienopyrrole solution. The 4H-dithienopyrrole solution was added dropwise into the round-bottom flask 1. After the dropwise addition, the reaction temperature was returned to room temperature to continue the reaction for 4 h. Then suction filtration was conducted, magnesium sulfate was used to remove water and dry filtrate, and the dried resultant was concentrated. A crude product was purified through a column, and a mobile phase was a mixed solution of ethyl acetate and n-hexane with a volume ratio of 1:8. 4H-dithienopyrrole propyl isopropyl sulfonate was obtained by purifying, with a structure shown as follows: 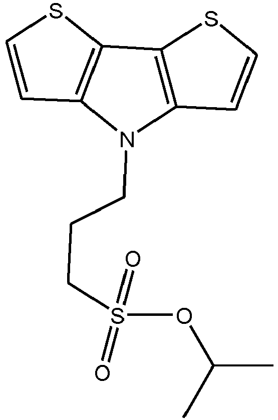
(3) In a round-bottom flask that was taken, the 4H-dithienopyrrole propyl isopropyl sulfonate was dissolved in ethanol. A vial was additionally taken to prepare a saturated sodium hydroxide solution. Sodium hydroxide was added dropwise into product 1 until the solution became cloudy and pH value was 11. The temperature was adjusted to 60°C. Stirring was continued for hydrolysis reaction for 3 h. Hydrochloric acid was then added dropwise to adjust the pH value. When system pH was 2, the dropwise addition of the hydrochloric acid was stopped. The ethanol in the system was removed by rotary evaporation. A small amount of water was added, and extracting was conducted with ethyl acetate. Oil phases were combined, and a product was dried and concentrated. A crude product was purified through a column. A mobile phase was a mixed solution of dichloromethane and n-hexane with a volume ratio of 1:8. Compound D was obtained by purifying.

The obtained target product D was subjected to a nuclear magnetic resonance test, and data were as follows:
1H NMR: δ 1.85 (1H, s), δ 2.09 (2H, tt, J = 6.7, 6.3 Hz), 3.48 (2H, t, J = 6.7 Hz), 3.95 (2H, t, J = 6.3 Hz), 7.08 (2H, d, J = 6.4 Hz), and 7.58 (2H, d, J = 6.4 Hz).

Other steps and conditions are the same as those in Example 1.

### Example 5

Example 5 was the same as Example 1, the only difference was that compound F was used in a fifth stage of step (2) to prepare a passivation layer, and the structure of the compound was shown as follows:

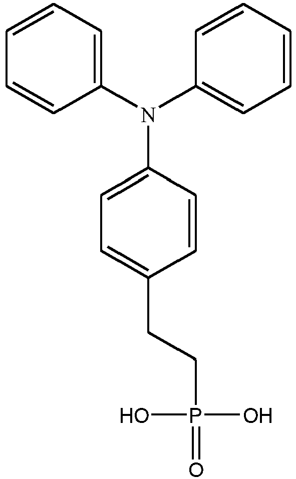

### Specific steps are as follows:

(1) To a clean and dry round-bottom flask that was taken, 60 ml diethyl phosphite (0.35 mol) and then 3.5 g 1-bromo-4-(2-bromoethyl) benzene (13.2 mmol) were added. A mixture was subjected to reflux heating for 24 hours. After cooling to room temperature, the mixture was dried and concentrated. A crude product was purified through a column, and a mobile phase was a mixed solution of ethyl acetate and n-hexane with a volume ratio of 1:4. An intermediate F-1 (diethyl [2-(4-bromophenyl) ethyl] phosphonate) was obtained by purifying, with a structure shown as follows: 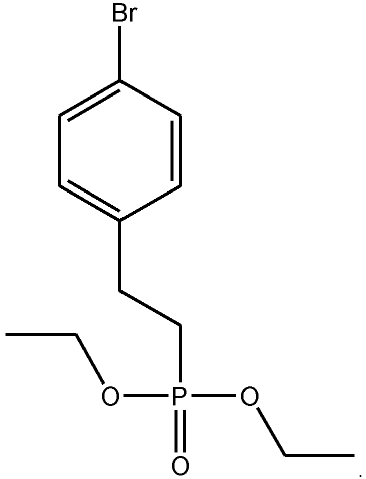
(2) In a clean and dry reaction flask, 9.6 g of the intermediate F-1 (29 mmol), 4.9 g of diphenylamine (29 mml), 0.5 g of Pd₂(dba)₃ (0.58 mmol), 2.316 mL of tri-tert-butylphosphine (2.32 mmol), and 18.86 g cesium carbonate (58 mmol) were mixed, and 100 mL of toluene was added. The reaction bottle was filled with a gas and vented three times through a double-row tube, and was sealed before an argon balloon was inserted. Reacting was allowed at 110°C for 8 h. After the reaction, the temperature was lowered to room temperature. After filtering, filtrate was dewatered, dried and concentrated to obtain a mixture. A crude product was purified through a column, and a mobile phase was a mixed solution of ethyl acetate and n-hexane with a volume ratio of 1:8. An intermediate F-2 was obtained by purifying, with a structure shown as follows: 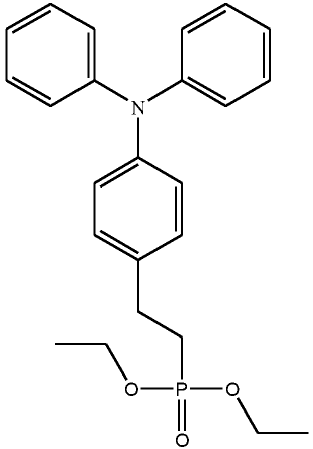
(3) To a clean and dry round-bottom flask that was taken, the intermediate F-2 was added. An argon atmosphere was maintained before 50 mL of 1,4-dioxane was added and 3.46 mL of bromotrimethylsilane (26 mmol) was dropwise added. The reaction was stirred at 25°C for reacting for 22 hours under the argon atmosphere. After the reaction was complete, methanol (3 ml) was added and stirring was continued for 3 h. Distilled water (10 ml) was dropwise added until the solution was opaque. The solution was stirred overnight, and a solid product was then filtered out, washed with water, and then dissolved in tetrahydrofuran until full dissolution. N-hexane was added until no more precipitation occurred. The solid product was filtered out and washed with n-hexane to obtain compound F.

The obtained target product F was subjected to a nuclear magnetic resonance test, and data were as follows:
1H NMR: δ 1.95 (2H, s), δ 2.52 (2H, t, J = 6.3 Hz), 2.90 (2H, t, J = 6.3 Hz), 6.40 (2H, ddd, J = 8.2, 1.3, 0.5 Hz), 7.00-7.36 (12H, 7.06 (tt, J = 7.8, 1.2 Hz), 7.14 (dtd, J = 8.2, 1.2, 0.5 Hz), 7.17 (ddd, J = 8.2, 1.6, 0.5 Hz), and 7.29 (dddd, J = 8.2, 7.8, 1.5, 0.5 Hz)).

Other steps and conditions are the same as those in Example 1.

### Example 6

Example 6 was the same as Example 1, the only difference was that compound G was used in a fifth stage of step (2) to prepare a passivation layer, and the structure of the compound was shown as follows:

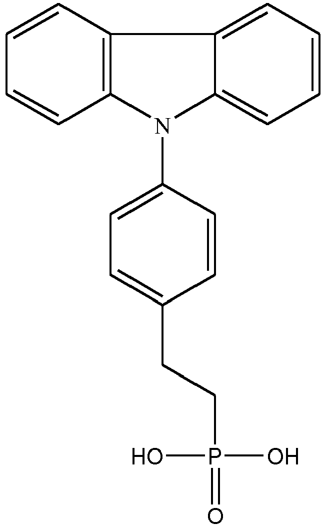

### Specific steps are as follows:

(1) Synthesis of diethyl [2-(4-bromophenyl) ethyl] phosphonate was the same as step (1) in Example 6.
(2) In a clean and dry reaction flask, 9.5 g of the diethyl [2-(4-bromophenyl) ethyl] phosphonate (29 mmol), carbazole (29 mml), 0.5 g of Pd₂(dba)₃ (0.58 mmol), 2.316 mL of tri-tert-butylphosphine (2.32 mmol), and 18.86 g cesium carbonate (58 mmol) were mixed, and 100 mL of toluene was added. The reaction bottle was filled with a gas and vented three times through a double-row tube, and was sealed before an argon balloon was inserted. Reacting was allowed at 110°C for 8 h. After the reaction, the temperature was lowered to room temperature. After filtering, filtrate was dewatered, dried and concentrated to obtain a mixture. A crude product was purified through a column, and a mobile phase was a mixed solution of ethyl acetate and n-hexane with a volume ratio of 1:8. An intermediate G-1 was obtained by purifying, with a structure shown as follows: 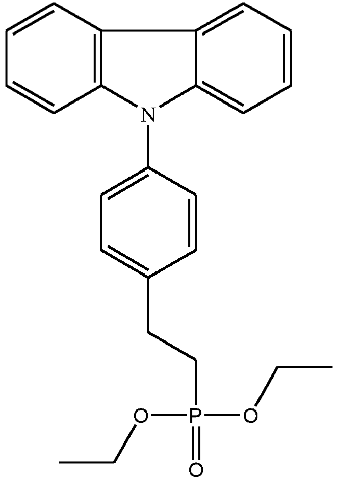
(3) To a clean and dry round-bottom flask that was taken, the intermediate G-1 was added. An argon atmosphere was maintained, 50 mL of 1,4-dioxane was added, and 3.46 mL of bromotrimethylsilane (26 mmol) was dropwise added. The reaction was stirred at 25°C for reacting for 22 hours under the argon atmosphere. After the reaction was complete, methanol (3 ml) was added and stirring was continued for 3 h. Distilled water (10 ml) was dropwise added until the solution was opaque. The solution was stirred overnight, and a solid product was then filtered out, washed with water, and then dissolved in tetrahydrofuran until full dissolution. N-hexane was added until no more precipitation occurred. The solid product was filtered out and washed with n-hexane to obtain compound G.

The obtained target product G was subjected to a nuclear magnetic resonance test, and data were as follows:
1H NMR: δ 1.83 (2H, s), δ 2.75 (2H, t, J = 6.0 Hz), 3.02 (2H, t, J = 6.0 Hz), 7.32-7.48 (4H, 7.39 (ddd, J = 8.0 , 6.2, 1.5 Hz), 7.41 (ddd, J = 8.4, 6.2, 2.6 Hz)), 7.57 (2H, ddd, J = 8.2, 1.6, 0.4 Hz), 7.71-7.90 (6H, 7.77 (ddd, J = 8.2, 1.5, 0.4 Hz), 7.82 (ddd, J = 8.4, 1.5, 0.5 Hz), and 7.83 (ddt, J = 8.0, 2.6, 0.5 Hz)).

Other steps and conditions are the same as those in Example 1.

### Example 7

Example 7 was the same as Example 1, the only difference was that following compound H was used in a fifth stage of step (2) to prepare a passivation layer:

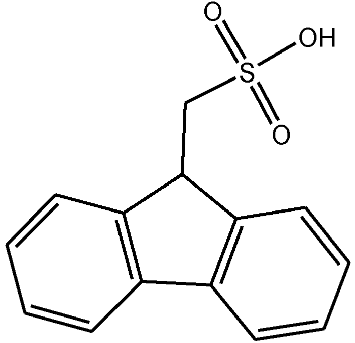

### Specific steps are as follows:

To a clean and dry round-bottom flask that was taken, 30 g (9H-fluoren-9-yl) chloromethane (0.140 mol), 300 mL acetone, and saturated Na₂SO₃ aqueous solution (300 ml) were added. A reflux condenser was provided. A mixture was stirred at 90°C for 8 hours, then cooled to ambient temperature, dried under vacuum at 30°C to remove acetone, and then 100 mL 6M hydrochloric acid solution was added under ice bath until pH value of the solution becomes about 1. At this point, white solid precipitated out, which was separated by filtration, and the solid was repeatedly washed with ether, and dried under vacuum at 40°C to obtain compound H.

The obtained target product H was subjected to a nuclear magnetic resonance test, and data were as follows:
1H NMR: δ 2.35 (1H, s), δ 3.98 (2H, d, J = 5.3 Hz), 4.91 (1H, t, J = 5.3 Hz), 7.29-7.43 (4H, 7.36 (ddd, J = 8.5 , 6.9, 1.4 Hz), 7.36 (ddd, J = 7.6, 6.9, 1.1 Hz)), 7.66 (2H, ddd, J = 7.6, 1.4, 0.5 Hz), and 7.83 (2H, ddd, J = 8.5, 1.1, 0.5 Hz).

Other steps and conditions are the same as those in Example 1.

### Example 8

Example 8 was substantially the same as Example 1, the only difference was that compound J was used in a fifth stage of step (2) to prepare a passivation layer, and the structure of the compound was shown as follows:

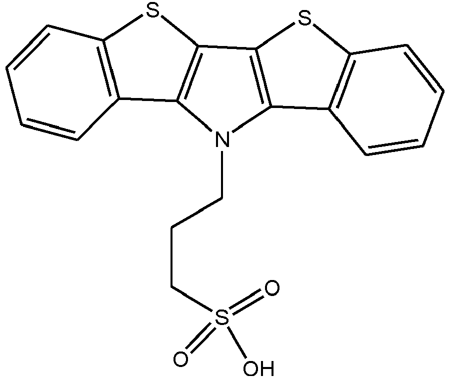

### Specific steps are as follows:

(1) To a clean and dry reaction flask, 2.94 g potassium carbonate (21.3 mmol), 1g tert-butyl carbamate (8.53 mmol), 2.08 g 2,3-dibromobenzo[b]thiophene (7.11 mmol), and 30 ml toluene solution were added. Under nitrogen current, 0.25 g N,N' dimethylethylenediamine (2.13 mmol) and 1.35 g copper iodide (7.11 mmol) were added, and stirred at 125°C for 12 hours. A reaction mixture was then cooled and subjected to suction filtering. Filtrate was dried over magnesium sulfate and then concentrated. A crude product was purified through a column, and a mobile phase was a mixed solution of dichloromethane and n-hexane with a volume ratio of 1:6. An intermediate J-1 was obtained by chromatographic purification, with a structure shown as follows: 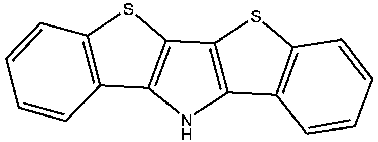
(2) 3.58 g (20 mmol) of 2-chloroethanesulfonyl chloride, 1.2 g (20 mmol) of isopropanol, and 40 ml of 1,3-dichloropropane were mixed in a round-bottom flask 1, and the temperature was lowered to 0°C. Another flask was taken. 5.59 g (20 mmol) of the intermediate J-1 was weighed and dissolved in 20 ml of 1,3-dichloropropane, and stirred sufficiently until full dissolution to obtain a solution of intermediate 1. The solution of intermediate 1 was added dropwise into the round-bottom flask 1. After the dropwise addition, the reaction temperature was returned to room temperature, and the reaction was continued for 4 h. After suction filtration, drying, and concentration, a crude product was purified through a column, and a mobile phase was a mixed solution of dichloromethane and n-hexane with a volume ratio of 1:8. An intermediate J-2 was obtained by purifying, with a structure shown as follows: 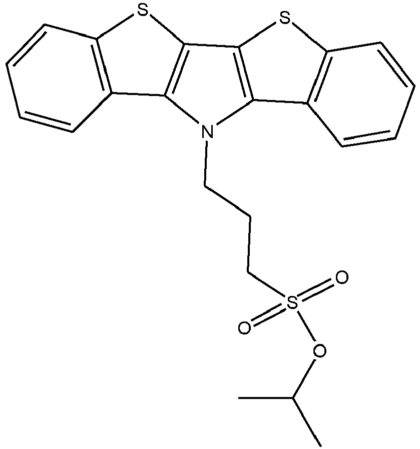
(3) In a round-bottom flask that was taken, the intermediate 2 was dissolved in ethanol. A vial was additionally taken to prepare a saturated sodium hydroxide solution. Sodium hydroxide was added dropwise into product 1 until the solution became cloudy and pH value was 11. The temperature was adjusted to 60°C. Stirring was continued for hydrolysis for 3 h to obtain hydrolysate, containing a compound with a structure shown as below:

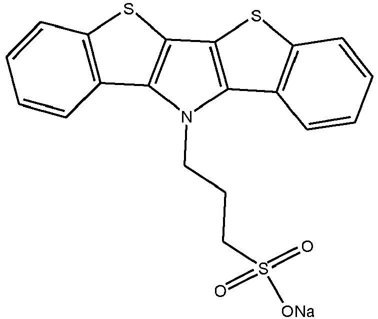

In the hydrolysate, hydrochloric acid was added dropwise to adjust the pH value. When pH was adjusted to 2, the dropwise addition was stopped. The ethanol in the system was removed by rotary evaporation. A small amount of water was added, and extracting was conducted with ethyl acetate. Oil phases were combined, and a product was dried and concentrated. A crude product was purified through a column. A mobile phase was a mixed solution of ethyl acetate and n-hexane with a volume ratio of 1:4. Compound J was obtained by purifying.

The obtained target product J was subjected to a nuclear magnetic resonance test, and data were as follows:
1H NMR: δ 1.83 (1H, s), δ 2.39 (2H, tt, J = 6.4, 6.3 Hz), 3.53 (2H, t, J = 6.4 Hz), 4.49 (2H, t, J = 6.3 Hz), 7.25 (2H, ddd, J = 8.5, 7.7, 1.2 Hz), 7.44 (2H, ddd, J = 7.8, 7.7, 1.5 Hz), 8.01-8.18 (4H, 8.08 (ddd, J = 7.8, 1.2, 0.5 Hz), and 8.11 (ddd, J = 8.5, 1.5, 0.5 Hz)).

Other steps and conditions are the same as those in Example 1.

### Example 9

Example 9 was substantially the same as Example 1, the only difference was that compound K was used in a fifth stage of step (2) to prepare a passivation layer, and the structure of the compound was shown as follows:

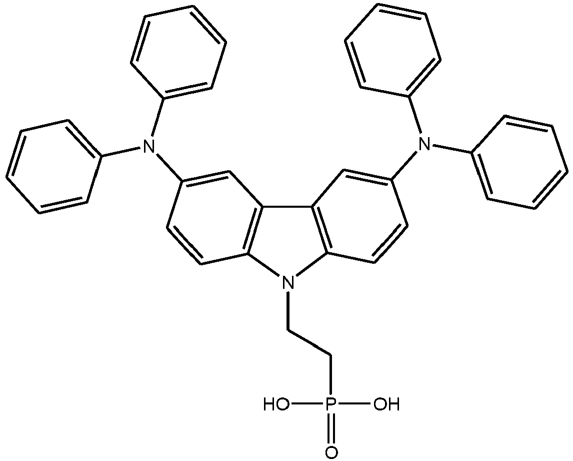

### Specific steps are as follows:

(1) N3,N3,N6,N6-tetraphenyl-9H-carbazole-3,6-diamine (CAS No. 608527-58-8, 20 mmol) was dissolved in 25 mL of 1,2-dibromoethane, and mixed evenly by stirring. 5 g of tetrabutylammonium bromide and 30 mL of 50wt% KOH aqueous solution were then added. The temperature was raised to 50°C. After stirring for 72 h, reaction was stopped. A product was extracted with dichloro. An organic phase was dried with anhydrous MgSO₄, and a crude product was obtained by rotary evaporation to remove the solvent. The crude product was purified through a column. A mobile phase was a mix of acetone and n-hexane with a volume ratio of 1: 10. An intermediate K-1 was obtained, with a structure shown as below: 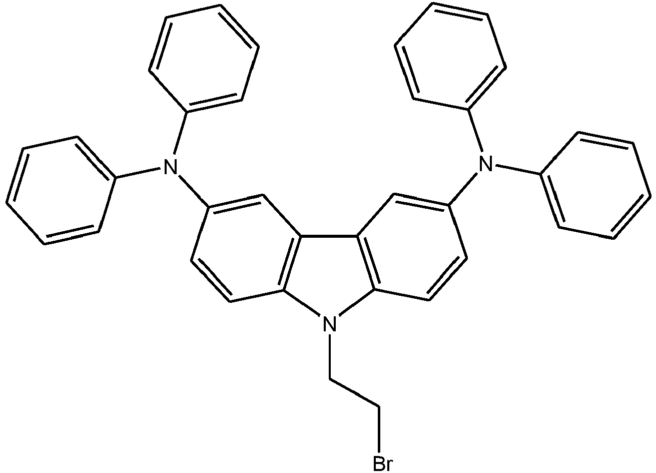
(2) Into a clean and dry round-bottom flask that was taken, 60 ml diethyl phosphite (0.35 mol) was added. 8.05 g of the intermediate K-1 was dissolved in triethyl phosphite. A mixture was subjected to reflux heating for 24 hours. After cooling to room temperature, the mixture was dried and concentrated. A crude product was purified through a column, and a mobile phase was a mixed solution of dichloromethane and n-hexane with a volume ratio of 1:2. An intermediate K-2 was obtained by purifying, with a structure shown as follows: 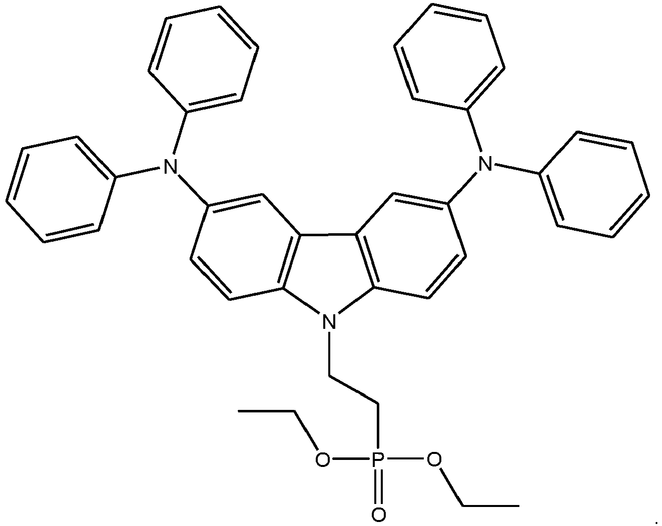
(3) To a clean and dry round-bottom flask that was taken, the intermediate K-2 was transferred. An argon atmosphere was maintained, and 50 mL 1,4-dioxane was added and 3.46 mL bromotrimethylsilane (26 mmol) was dropwise added. Stirring was conducted at 25°C for reacting for 22 hours under the argon atmosphere. Methanol (3 ml) was added and stirring was continued for 3 h. Distilled water (10 ml) was dropwise added until the solution was opaque. The solution was stirred overnight, and a solid product was then filtered out, washed with water, and then dissolved in tetrahydrofuran until full dissolution. N-hexane was added until no more precipitation occurred. The precipitation was filtered out, and washed with n-hexane to obtain compound K.

The obtained target product K was subjected to a nuclear magnetic resonance test, and data were as follows:
1HNMR: δ 1.96 (2H, s), δ 2.72 (2H, t, J = 7.0 Hz), 4.41 (2H, t, J = 7.0 Hz), 6.70 (2H, dd, J = 8.3, 1.8 Hz), 6.84 (8H, dtd, J = 8.2, 1.2, 0.5 Hz), 7.03 (4H, tt, J = 7.8, 1.2 Hz), 7.29 (8H, dddd, J = 8.2, 7.8, 1.8, 0.5 Hz), 7.49 (2H, dd, J = 8.3, 0.4 Hz), and 7.63 (2H, dt, J = 1.8, 0.5 Hz).

Other steps and conditions are the same as those in Example 1.

### Example 10

Example 10 was substantially the same as Example 1, the only difference was that compound L was used in a fifth stage of step (2) to prepare a passivation layer, and the structure of the compound was shown as follows:

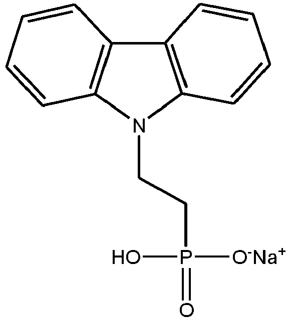

### Specific steps are as follows:

To a clean and dry round-bottom flask that was taken, 5.5 g compound A (20 mmol) was added, and dissolved in 20 mL of absolute ethanol. Another 0.8 g of sodium hydroxide (20 mmol) was dissolved in 20 mL of water to obtain a sodium hydroxide solution. The sodium hydroxide solution was added dropwise into 2-(9H-carbazole-9-ethyl) phosphonic acid. Ethanol was removed by rotary evaporation. Water and ethyl acetate were added. An oil phase was removed. Ethanol was added into a water phase. Concentrating was conducted to remove the solvent before drying under vacuum at 60°C to give compound L as a product.

The obtained target product L was subjected to a nuclear magnetic resonance test, and data were as follows:
1H NMR: δ 1.85 (1H, s), δ 2.36-2.48 (2H, 2.42 (t, J = 6.1 Hz), 2.42 (t, J = 6.1 Hz)), 4.15-4.27 (2H, 4.21 (t, J = 6.1 Hz), 4.21 (t, J = 6.1 Hz)), 7.13 (2H, ddd, J = 8.0, 7.6, 2.3 Hz), 7.30 (2H, ddd, J = 8.0, 7.6, 1.6 Hz), 7.52 (2H , ddt, J = 8.0, 2.3, 0.5 Hz), and 7.68 (2H, ddd, J = 8.0, 1.6, 0.5 Hz).

### Example 11

Example 11 was the same as Example 1, the only difference was that compound M was used in a fifth stage of step (2) to prepare a passivation layer, and the structure of the compound was shown as follows:

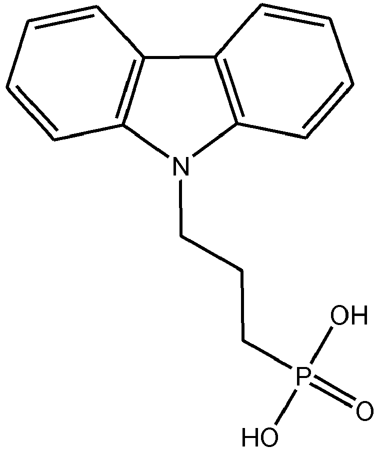

### Specific steps are as follows:

(1) This step was same as step (1) in Example 1, only except that the 1,2-dibromoethane in Example 1 was replaced with 1,2-dibromoethane in an equimolar amount.

### Step (2) to step (3) refer to step (2) to step (3) of Example 1.

The obtained target product M was subjected to a nuclear magnetic resonance test, and data were as follows:
1HNMR: δ 1.83(2H,s), δ 2.09 (2H, tt, J = 7.4, 6.3 Hz), 2.61 (2H, t, J = 7.4 Hz), 4.39 (2H, t, J = 6.3 Hz), 7.13 (2H, ddd, J = 8.0, 7.6, 2.3 Hz), 7.30 (2H, ddd, J = 8.0, 7.6, 1.6 Hz), 7.52 (2H, ddt, J = 8.0, 2.3, 0.5 Hz), and 7.68 (2H, ddd, J = 8.0, 1.6, 0.5 Hz).

### Example 12

Example 12 was the same as Example 1, the only difference was that compound N was used in a fifth stage of step (2) to prepare a passivation layer, and the structure of the compound was shown as follows:

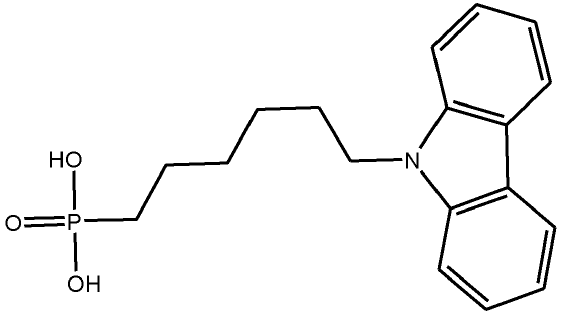

### Specific steps are as follows:

(1) This step was same as step (1) in Example 1, only except that the 1,2-dibromoethane in Example 1 was replaced with 1,6-dibromohexane in an equimolar amount.

Step (2) to step (3) refer to step (2) to step (3) of Example 1.

The obtained target product N was subjected to a nuclear magnetic resonance test, and data were as follows:
1H NMR: δ 1.77 (2H, s), δ 1.18-1.37 (4H, 1.24 (tt, J = 7.7, 7.2 Hz), 1.30 (tt, J = 7.2, 6.5 Hz)), 1.73 (2H, tt, J = 7.7, 7.4 Hz), 2.06 (2H, tt, J = 6.5, 6.3 Hz), 2.56 (2H, t, J = 7.4 Hz), 4.44 (2H, t, J = 6.3 Hz), 7.17-7.31 (4H, 7.24 (ddd, J = 8.0, 7.8, 1.8 Hz), 7.24 (ddd, J = 8.0, 7.8, 1.6 Hz)), 7.52 (2H, ddt, J = 8.0, 1.8, 0.5 Hz), and 7.74 (2H, ddd , J = 8.0, 1.6, 0.5 Hz).

### Example 13

Example 13 was the same as Example 1, the only difference was that compound P was used in a fifth stage of step (2) to prepare a passivation layer, and the structure of the compound was shown as follows:

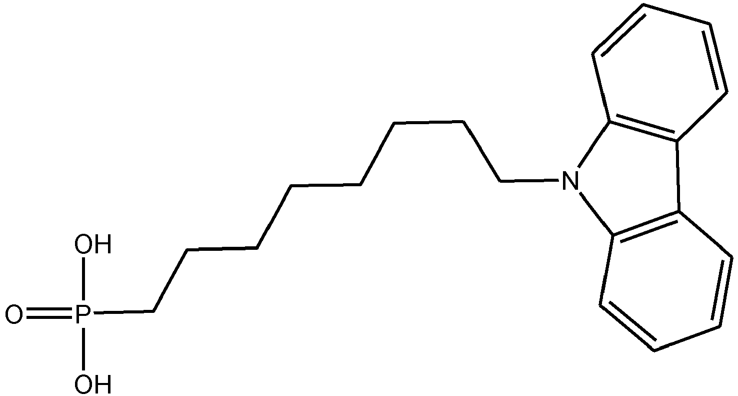

### Specific steps are as follows:

(1) This step was same as step (1) in Example 1, only except that the 1,2-dibromoethane in Example 1 was replaced with 1,8-dibromooctane in an equimolar amount.

Step (2) to step (3) refer to step (2) to step (3) of Example 1.

The obtained target product P was subjected to a nuclear magnetic resonance test, and data were as follows:
1H NMR: δ 1.75 (2H, s), δ 1.18-1.37 (8H, 1.25 (tt, J = 7.1, 6.9 Hz), 1.26 (tt, J = 6.9, 6.5 Hz), 1.25 (quint, J = 6.5 Hz), 1.30 (tt, J = 6.6, 6.5 Hz)), 1.73 (2H, tt, J = 7.4, 7.1 Hz), 2.06 (2H, tt, J = 6.6, 6.3 Hz), 2.62 (2H, t, J = 7.4 Hz), 4.44 (2H, t, J = 6.3 Hz), 7.17-7.31 (4H, 7.24 (ddd, J = 8.0, 7.8, 1.8 Hz), 7.24 (ddd, J = 8.0, 7.8, 1.6 Hz )), 7.52 (2H, ddt, J = 8.0, 1.8, 0.5 Hz), and 7.74 (2H, ddd, J = 8.0, 1.6, 0.5 Hz).

### Comparative Example 1

Comparative Example 1 was substantially the same as Example 1, the only difference was that a compound used in a fifth stage of step (2) to prepare a passivation layer has a structure shown as follows:

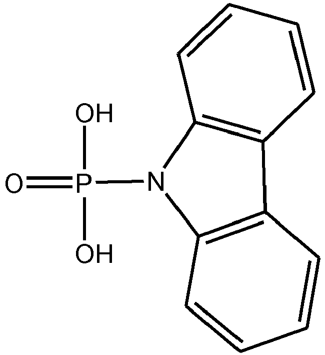

### Specific steps are as follows:

(1) Synthesis of N-bromocarbazole refers to patent CN100368399, and details will not be repeated here.
(2) Synthesis of diethyl 9H-carbazole-9-ylphosphonate

4.9 g of N-bromocarbazole (20 mml) was dissolved in 15 mL of diethyl phosphite (87.5 mmol), subjected to heating reflux, and reacted for 24 h. After reaction, most of the organic solvent was removed by rotary evaporation. A product was purified through a column. A mobile phase was a mixed solution of acetone and n-hexane with a volume ratio of 1:4. An intermediate with a structure shown as below was obtained:

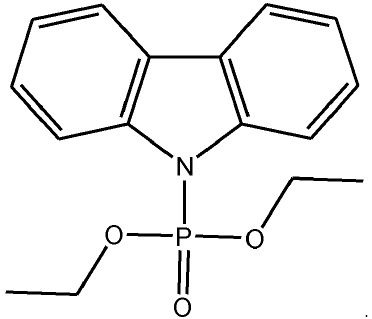

(3) The intermediate obtained in (2) was dissolved in 20 mL ethanol. NaOH saturated solution was added to adjust pH value to 11, and reacted at 60°C for 24 h, and hydrochloric acid was then dropwise added to adjust pH to 2. The solvent was removed by rotary evaporation after reaction. The resultant was then dissolved in dichloromethane, washed with saturated brine, and then dried with anhydrous magnesium sulfate. Most of the solvent was removed by concentrating to obtain a crude product. The crude product was purified through a column. A mobile phase was a mixed solution of ethyl acetate and n-hexane with a volume ratio of 1:4. A target compound was obtained. The obtained target product was subjected to a nuclear magnetic resonance test, and data were as follows:
1HNMR: δ 2.65 (2H, s), 7.31-7.50 (4H,7.38 (ddd, J= 8.5, 6.9, 1.5 Hz), 7.43 (ddd, J= 8.5, 6.9, 1.4 Hz)), 7.57 (2H, ddd, J = 8.5, 1.5, 0.5 Hz), and 7.84 (2H, ddd, J = 8.5, 1.4, 0.5 Hz).

Other steps are the same as those in Example 1, and specific results are shown in table 1.

### Comparative Example 2

Comparative Example 2 was substantially the same as Example 1, and the only difference was that a self-assembled compound used in preparing a passivation layer was pyridine p-toluenesulfonate.

Other steps are the same as those in Example 1, and specific results are shown in table 1.

### Comparative Example 3

Comparative Example 3 was substantially the same as Example 1, and the only difference was that a fifth stage in step (2) was omitted, that is, a perovskite layer was directly prepared on a nickel oxide substrate.

Other steps are the same as those in Example 1, and specific results are shown in table 1.

### Comparative Example 4

Comparative Example 4 was substantially the same as Example 1, and the only difference was that polystyrene was used in a fifth stage of step (2) to prepare a passivation layer.

Other steps are the same as those in Example 1, and specific results are shown in table 1.

**Table 1**

| | Day 3 Efficiency | Day 30 Efficiency |
|---|---|---|
| Example 1 | 18.01% | 18.11% |
| Example 2 | 18.23% | 18.26% |
| Example 3 | 18.42% | 18.58% |
| Example 4 | 18.50% | 18.66% |
| Example 5 | 18.44% | 18.59% |
| Example 6 | 18.15% | 18.19% |
| Example 7 | 18.28% | 18.33% |
| Example 8 | 18.66% | 18.70% |
| Example 9 | 18.86% | 19.02% |
| Example 10 | 18.45% | 18.52% |
| Example 11 | 18.25% | 18.32% |
| Example 12 | 18.30% | 18.32% |
| Example 13 | 18.02% | 18.01% |
| Comparative Example 1 | 17.85% | 17.88% |
| Comparative Example 2 | 17.55% | 17.48% |
| Comparative Example 3 | 17.15% | 15.88% |
| Comparative Example 4 | 17.23% | 16.25% |

Finally, it should be noted that the above Examples are merely used for illustrating rather than limiting the technical solutions of the present application. Although the present application has been described in detail with reference to the above various Examples, those of ordinary skill in the art should understand that the technical solutions specified in the above various Examples may still be modified, or some or all of the technical features therein may be equivalently substituted; and such modifications or substitutions do not make the essence of the corresponding technical solutions depart from the scope of the technical solutions of the various Examples of the present application, which shall fall within the scope of the claims and the specification of the present application. In particular, the technical features mentioned in the various Examples may be combined in any manner as long as there is no structural conflict. The present application is not limited to the specific Examples disclosed herein, but rather includes all technical solutions falling within the scope of the claims.

## Claims

1. An organic compound, shown as in formula (1): 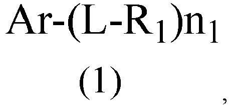
wherein Ar is selected from any one of substituted or unsubstituted aryl with 6 to 50 ring atoms, substituted or unsubstituted heteroaryl with 5 to 50 ring atoms, and arylamino as shown in formula (A): 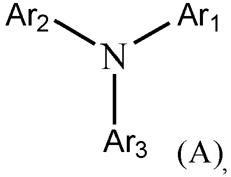
L is selected from acyclic alkylene with 1 to 10 carbon atoms; R₁ is an oxyacid group;
Ar₁ to Ar₃ are independently selected from any one of H, substituted or unsubstituted alkenyl with 2 to 30 carbon atoms, and substituted or unsubstituted aryl with 7 to 30 carbon atoms, respectively, and at least one of Ar₁ to Ar₃ is selected from substituted or unsubstituted aryl with 7 to 30 carbon atoms;
n₁ is selected from any integer from 1 to 3;
or the organic compound is an oxyacid radical salt of the compound as shown in formula (1).

2. The organic compound of claim 1, wherein L is selected from acyclic alkylene with 3 to 11 carbon atoms.

3. The organic compound of claim 1, wherein the acyclic alkylene is linear alkylene.

4. The organic compound of any one of claims 1 to 3, wherein R₁ is, at each occurrence, independently selected from any one of a phosphonic acid group, a sulfonic acid group, a carboxylic acid group, a sulfinic acid group, a boric acid group, or a silicic acid group, respectively.

5. The organic compound of any one of claims 1 to 3, wherein R₁ is, at each occurrence, independently selected from any one of the following structures, respectively: 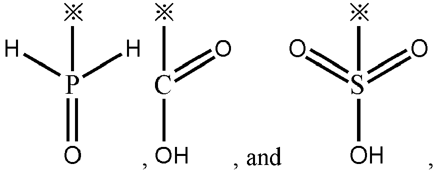 and 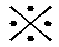 represents a linking site.

6. The organic compound of any one of claims 1 to 3, wherein heteroatoms in the substituted or unsubstituted heteroaryl with 5 to 50 ring atoms are selected from at least one of N, O, and S.

7. The organic compound of any one of claims 1 to 3, wherein Ar is selected from any one of formulas (A) to (C): 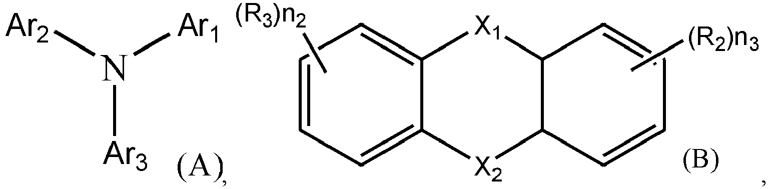 and 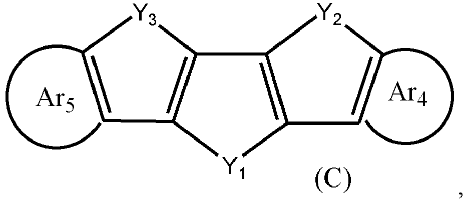
wherein X₁ and X₂ are independently selected from any one of a single bond, C(R₄R₅), O, S, N, NR₄, C=O, or S=O, respectively, and X₁ and X₂ are not single bonds at the same time;
Y₁, Y₂, and Y₃ are independently selected from any one of C(R₄R₅), O, S, N, NR₄, C=O, or S=O, respectively;
R₂ to R₅ are, at each occurrence, independently selected from any one of H, D, a halogen group, -N(R₆)₂, -CONR₆, -OCOR₆, substituted or unsubstituted alkyl with 1 to 30 carbon atoms, substituted or unsubstituted aryl with 6 to 30 carbon atoms, and substituted or unsubstituted heteroaryl with 5 to 30 carbon atoms, respectively;
R₆ is, at each occurrence, independently selected from any one of H, D, substituted or unsubstituted alkyl with 1 to 30 carbon atoms, substituted or unsubstituted alkenyl with 2 to 30 carbon atoms, substituted or unsubstituted aryl with 6 to 30 carbon atoms, and substituted or unsubstituted heteroaryl with 5 to 30 carbon atoms, respectively;
Ar₄ and Ar₅ are independently selected from any one of H, substituted or unsubstituted aryl with 6 to 30 carbon atoms, and substituted or unsubstituted heteroaryl with 5 to 30 carbon atoms, respectively; and
n₂ and n₃ are independently selected from any integer from 1 to 4, respectively, with 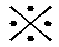 representing a linking site.

8. The organic compound of claim 7, wherein X₁ and X₂ are independently selected from any one of a single bond, C(R₄R₅), O, S, N, NR₄, respectively, and X₁ and X₂ are not single bonds at the same time; and
Y₁, Y₂, and Y₃ are independently selected from any one of C(R₄R₅), O, S, and N, respectively.

9. The organic compound of claim 8, wherein R₂ to R₅ are, at each occurrence, independently selected from any one of H, D, a halogen group, amino, -CONR₆, -OCOR₆, unsubstituted alkyl with 1 to 5 carbon atoms, halogen- or carboxy-substituted alkyl with 1 to 5 carbon atoms, unsubstituted aryl with 6 to 15 carbon atoms, halogen-substituted aryl with 6 to 15 carbon atoms, unsubstituted heteroaryl with 6 to 15 carbon atoms, and halogen-substituted heteroaryl with 5 to 15 carbon atoms, respectively; and
R₆ is, at each occurrence, independently selected from any one of H, D, substituted or unsubstituted alkyl with 1 to 5 carbon atoms, substituted or unsubstituted alkenyl with 2 to 6 carbon atoms, substituted or unsubstituted aryl with 6 to 15 carbon atoms, and substituted or unsubstituted heteroaryl with 5 to 15 carbon atoms, respectively.

10. The organic compound of claim 7, wherein R₄ in NR₄ is, at each occurrence, independently selected from any one of H, D, substituted or unsubstituted alkyl with 1 to 5 carbon atoms, and the following structures, respectively: 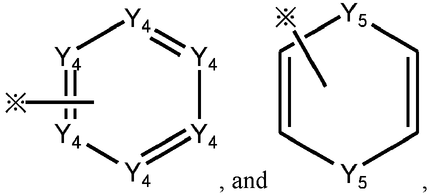
Y₄ is, at each occurrence, independently selected from CR₉ or N, respectively, and Y₄ in the same structural formula is not N at the same time;
Y₅ is, at each occurrence, independently selected from CR₉R₁₀ or NR₉, respectively;
R₉ to R₁₀ are, at each occurrence, independently selected from any one of H, D, substituted or unsubstituted linear alkyl with 1 to 10 carbon atoms, substituted or unsubstituted branched alkyl with 3 to 20 carbon atoms and substituted or unsubstituted cycloalkyl with 3 to 20 carbon atoms, respectively; and
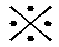 represents a linking site with N in NR₄.

11. The organic compound of claim 7, wherein Ar₄ and Ar₅ are independently selected from any one of H and the following structures, respectively: 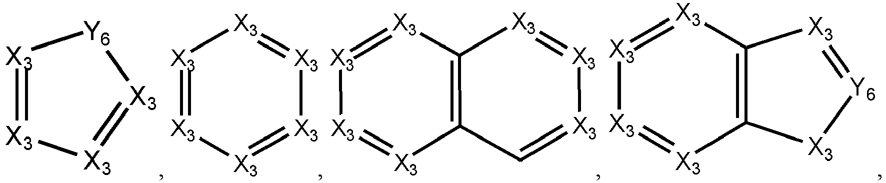 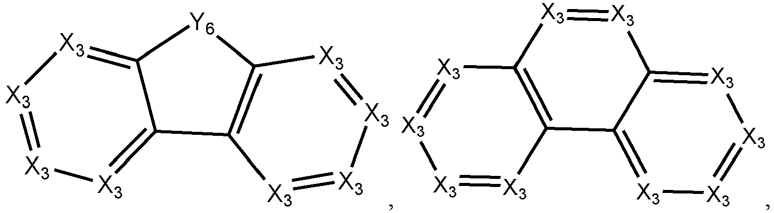 and 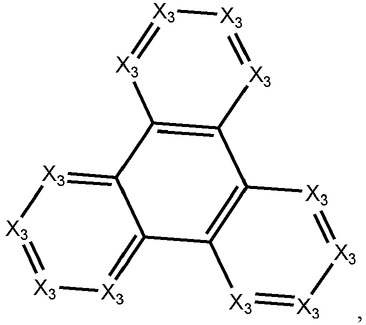
wherein: Y₆ is selected from any one of CR₇R₈, O, S, S=O, and C=O;
X₃ is, at each occurrence, independently selected from CR₇ or N, respectively, and X₃ in the same structural formula is not N at the same time; and
R₇ and R₈ are, at each occurrence, independently selected from any one of H, D, substituted or unsubstituted linear alkyl with 1 to 10 carbon atoms, substituted or unsubstituted branched alkyl with 3 to 20 carbon atoms and substituted or unsubstituted cycloalkyl with 3 to 20 carbon atoms, respectively.

12. The organic compound of claim 7, wherein Ar is selected from any one of the following structures: 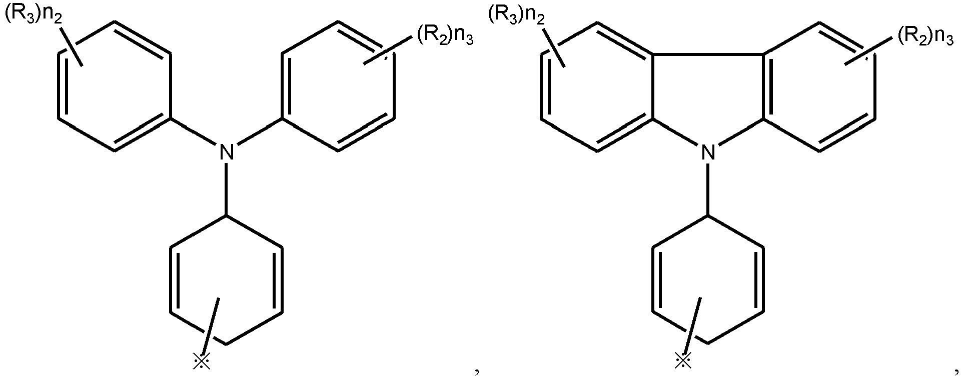 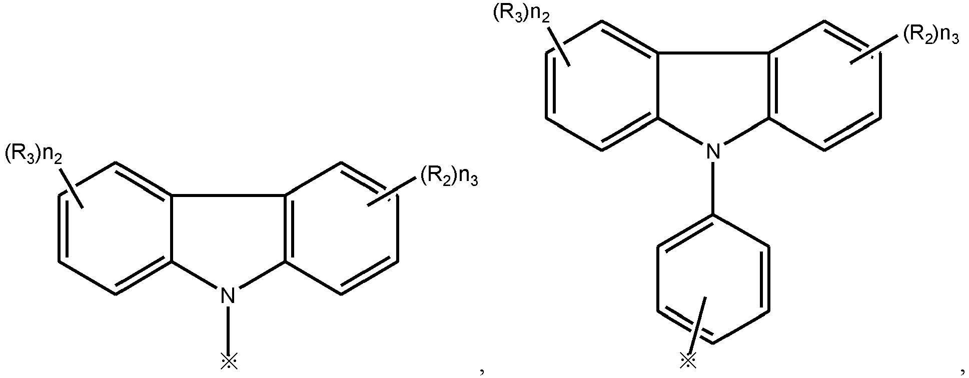 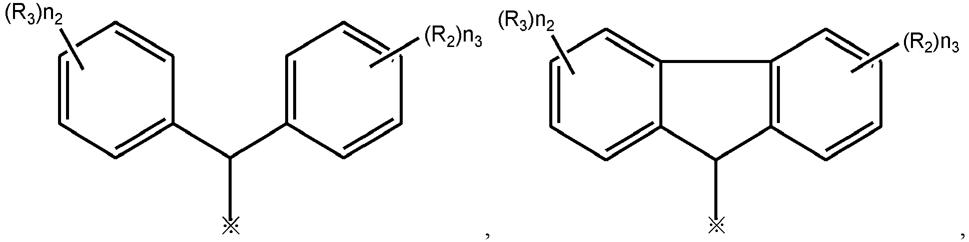 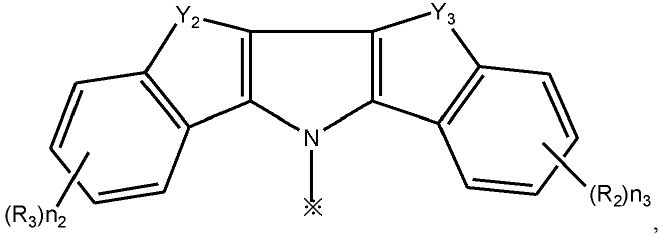 and

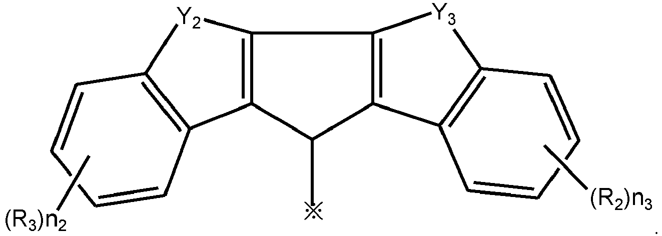

13. The organic compound of any one of claims 1 to 3, wherein the oxyacid radical salt of the compound as shown in formula (1) comprises an anion and a cation, the anion is formed by at least one alcoholic hydroxyl in an oxyacid group in the compound as shown in formula (1) losing H, and the cation is selected from a metal ion or NH₄⁺.

14. Use of an organic compound as a metal deactivator, the organic compound being as shown in formula (2): 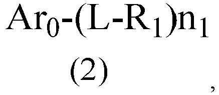
wherein Ar₀ is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group;
n₁ is selected from any integer from 1 to 3;
or the organic compound is an oxyacid radical salt of the compound as shown in formula (2).

15. Use of an organic compound in preparing a passivation film, the organic compound being as shown in formula (2): 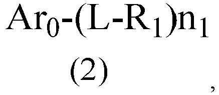
wherein Ar₀ is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group;
n₁ is selected from any integer from 1 to 3;
or the organic compound is an oxyacid radical salt of the compound as shown in formula (2).

16. A passivation film, wherein components of the passivation film comprise an organic compound as shown in formula (2) and/or an oxyacid radical salt of the compound as shown in formula (2): 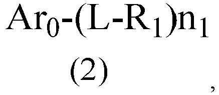
wherein Ar₀ is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group; and
n₁ is selected from any integer from 1 to 3.

17. A method of preparing a passivation film of claim 16, comprising the following steps:
providing a substrate;
mixing an organic compound as shown in formula (2) and/or an oxyacid radical salt of the compound as shown in formula (2) and a solvent to prepare a mixed solution;
and coating a surface of the substrate with the mixed solution, and drying to obtain the passivation film.

18. A solar battery, comprising a hole transport layer and a passivation layer that are stacked, wherein components of the hole transport layer comprise a metal oxide, and the passivation layer comprises an organic compound as shown in formula (2) and/or an oxyacid radical salt of the compound as shown in formula (2): 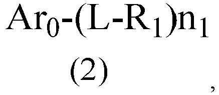
wherein Ar₀ is selected from any one of substituted or unsubstituted aryl with 6 to 50 ring atoms, substituted or unsubstituted heteroaryl with 5 to 50 ring atoms, and substituted or unsubstituted arylamino with 6 to 50 ring atoms;
L is selected from acyclic alkylene with 1 to 10 carbon atoms;
R₁ is an oxyacid group; and
n₁ is selected from any integer from 1 to 3.

19. The solar battery of claim 18, wherein the solar battery further comprises a perovskite layer, and the perovskite layer is located on a surface of the passivation layer away from the hole transport layer; and/or
components of the hole transport layer comprise at least one of nickel oxide and zinc oxide.

20. The solar battery of any one of claims 18 to 19, wherein the passivation layer is 0.1 nm to 10 nm in thickness.

21. An electrical apparatus, comprising a solar battery of any one of claims 18 to 20.
